(19) Europäisches Patentamt
European Patent Office
Office européen des brevets

(11) EP 2 411 383 B1

(12) FASCICULE DE BREVET EUROPEEN

(45) Date de publication et mention de la délivrance du brevet:
**22.10.2014 Bulletin 2014/43**

(21) Numéro de dépôt: **10715985.7**

(22) Date de dépôt: **25.03.2010**

(51) Int Cl.:
***C07D 409/06*** (2006.01) ***C07D 409/12*** (2006.01)
***A61K 31/4535*** (2006.01) ***A61P 25/00*** (2006.01)

(86) Numéro de dépôt international:
**PCT/FR2010/050551**

(87) Numéro de publication internationale:
**WO 2010/109150 (30.09.2010 Gazette 2010/39)**

# (54) DERIVES DE 3-ALCOXY-4,5-DIARYLTHIOPHENE-2-CARBOXAMIDE, LEUR PREPARATION ET LEUR APPLICATION EN THERAPEUTIQUE

DERIVATE VON 3-ALKOXY-4,5-DIARYLTHIOPHEN-2- CARBONSÄUREAMID, DEREN HERSTELLUNG UND DEREN THERAPEUTISCHE VERWENDUNG

DERIVATIVES OF 3-ALKOXY-4,5-DIARYLTHIOPHENE-2-CARBOXAMIDE, PREPARATION THEREOF, AND THERAPEUTIC USE THEREOF

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA ME RS**

(30) Priorité: **27.03.2009 FR 0901465**

(43) Date de publication de la demande:
**01.02.2012 Bulletin 2012/05**

(73) Titulaire: **SANOFI**
**75008 Paris (FR)**

(72) Inventeurs:
- **DUCOUX, Jean-Philippe**
**F-75013 Paris (FR)**
- **RINALDI-CARMONA, Murielle**
**F-75013 Paris (FR)**
- **ROUQUETTE, Arnaud**
**F-75013 Paris (FR)**

(74) Mandataire: **Werner, Alain Henri et al**
**Sanofi**
**Département Brevets**
**54, rue La Boétie**
**75008 Paris (FR)**

(56) Documents cités:
**WO-A-2004/099157** **WO-A-2005/035488**
**WO-A-2007/046550**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet des dérivés de 3-alkoxy-4,5-diarylthiophène-2-carboxamide, leur préparation et leur application en thérapeutique.

Des dérivés de diphénylpyrazole, présentant une affinité pour les récepteurs $CB_1$ des cannabinoïdes ont été décrits notamment dans les brevets US 5 624 941, EP 0 576 357, EP 0 656 354 et EP 1 150 961.

Des dérivés de 5,6-diphényl-2-pyrazine-carboxamide sont décrits dans la demande internationale WO 03/051 850 comme des antagonistes des récepteurs $CB_1$.

Des dérivés de 1,2-diphényl-4-imidazolecarboxamide sont décrits dans la demande de brevet internationale WO 03/027 076 comme des agonistes des récepteurs $CB_1$, des agonistes partiels ou des antagonistes.

Des dérivés de 4,5-diarylthiophène ayant des propriétés analgésiques sont décrits dans la demande internationale WO 91/19 708.

D'autres dérivés de 4,5-diarylthiophène sont décrits dans les demandes internationales WO 2005/035 488, WO 2006/070 106, WO 2006/077 320 et WO 2006/084 975 comme des antagonistes des récepteurs $CB_1$.

On a maintenant trouvé des nouveaux dérivés de 3-alcoxy-4,5-diarylthiophène-2-carboxamide portant un substituant particulier en position 3 du noyau thiophène qui possèdent des propriétés antagonistes des récepteurs $CB_1$ des cannabinoïdes. En particulier ces nouveaux dérivés ont des propriétés antagonistes des récepteurs $CB_1$ périphériques et présentent une faible pénétration au niveau du cerveau.

Ainsi la présente invention a pour objet des composés répondant à la formule (I) :

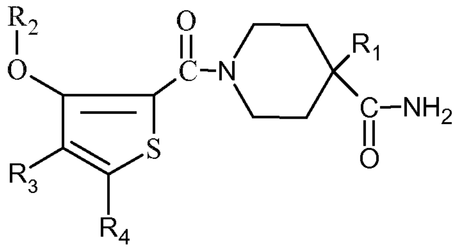

(I)

dans laquelle :

- $R_1$ représente :

  . un groupe $-NR_5R_6$ ;
  . un phényle non substitué ou substitué une ou plusieurs fois par des substituants choisis indépendamment parmi un atome d'halogène, un $(C_1\text{-}C_4)$alkyle, un $(C_1\text{-}C_4)$alcoxy, un trifluorométhyle ;

- $R_2$ représente :

  . un $(C_1\text{-}C_4)$alkyle ;
  . un groupe $-X\text{-}R_7$ ;

- $R_3$ et $R_4$ représentent chacun indépendamment un phényle substitué une ou plusieurs fois par un substituant choisi indépendamment parmi un atome d'halogène, un $(C_1\text{-}C_4)$alkyle, un $(C_1\text{-}C_4)$alcoxy, un trifluorométhyle ; :
- $R_5$ représente un atome d'hydrogène ou un $(C_1\text{-}C_4)$alkyle ;
- $R_6$ représente un $(C_1\text{-}C_4)$alkyle non substitué ou substitué par un ou plusieurs atomes de fluor ou par un phényle non susbtitué ou substitué par un atome d'halogène ;
- ou bien $R_5$ et $R_6$ ensemble avec l'atome d'azote auquel ils sont liés constituent un hétérocycle choisi parmi : l'azétidine, la pyrrolidine, la pipéridine, ledit hétérocycle étant non substitué ou subtitué une ou plusieurs fois par un atome d'halogène ;
- X représente un $(C_1\text{-}C_5)$alkylène ;
- $R_7$ représente un groupe $-OR_8$, un groupe $-NR_9R_{10}$, un groupe $-SO_2\text{-}(C_1\text{-}C_4)$alkyle ;
- $R_8$ représente un atome d'hydrogène ou un $(C_1\text{-}C_4)$alkyle ;
- $R_9$ représente un atome d'hydrogène ou un $(C_1\text{-}C_4)$alkyle ;
- $R_{10}$ représente un atome d'hydrogène, un groupe $-COR_{11}$, un groupe $-SO_2R_{11}$ ou un groupe $-CO(CH_2)_mOH$ ;
- $R_{11}$ représente un $(C_1\text{-}C_4)$alkyle non substitué ou substitué par un ou plusieurs atomes de fluor ;
- m représente 1, 2 ou 3.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Par atome d'halogène on entend un atome de brome, de chlore, de fluor ou d'iode.

Par $(C_1-C_4)$alkyle on entend un radical alkyle linéaire ou ramifié de un à quatre atomes de carbone, tel que le radical méthyle, éthyle, propyle, isopropyle, butyle, isobutyle, *sec*-butyle, *tert*-butyle.

Par $(C_1-C_5)$alkylène on entend un radical bivalent de deux à cinq atomes de carbone tel que le radical méthylène, éthylène, triméthylène, tétraméthylène ou le radical pentaméthylène.

Par $(C_1-C_4)$alcoxy on entend un radical alcoxy linéaire ou ramifié de un à quatre atomes de carbone tel que le radical méthoxy, éthoxy, propoxy, isopropoxy, butoxy, *sec*-butoxy, *tert*-butoxy.

Par $(C_3-C_7)$cycloalkyle on entend un radical carboné cyclopropyle, cyclobutyle, cyclopentyle, cyclohexyle ou cycloheptyle.

Selon la présente invention on préfère les composés de formule (I) dans laquelle :

- $R_1$ représente :
  . un groupe $-NR_5R_6$ ;
  . un phényle ;
- $R_2$ représente :
  . un méthyle ;
  . un groupe $-X-R_7$ ;
- $R_3$ et $R_4$ représentent chacun indépendamment un phényle substitué une ou deux fois par un atome d'halogène ;
- $R_5$ représente un atome d'hydrogène ;
- $R_6$ représente un $(C_1-C_4)$alkyle substitué par un ou plusieurs atomes de fluor ou par un phényle non substitué ou substitué par un atome d'halogène ;
- ou bien $R_5$ et $R_6$ ensemble avec l'atome d'azote auquel ils sont liés constituent un radical pipéridin-1-yle non substitué ou substitué une ou deux fois par un atome d'halogène ;
- X représente un $(C_1-C_3)$alkylène ;
- $R_7$ représente un groupe $-OR_8$, un groupe $-NR_9R_{10}$, un groupe $-SO_2-CH_3$ ;
- $R_8$ représente un atome d'hydrogène ;
- $R_9$ représente un atome d'hydrogène ;
- $R_{10}$ représente un atome d'hydrogène, un groupe $-CO-CH_3$, un groupe $-SO_2-CH_3$ ou un groupe $-COCH_2OH$ ;

à l'état de base ou de sel d'addition à un acide.

Particulièrement, on préfère les composés de formule (I) dans laquelle :

- $R_1$ représente :
  . un groupe $-NH(CH_2)_2-CF_3$, un groupe 4-fluorobenzylamino, un groupe benzylamino, un radical pipéridin-1-yle, un radical 4,4-difluoropipéridin-1-yle ;
  . un phényle ;
- $R_2$ représente :
  . un méthyle ;
  . un groupe $-X-R_7$ ;
- $R_3$ représente un 4-chlorophényle ;
- $R_4$ représente un 2-chlorophényle, un 2,4-dichlorophényle ;
- X représente un méthylène, un éthylène, un triméthylène ;
- $R_7$ représente un radical -OH, un radical $-NH_2$, un groupe $-NHCOCH_3$, un groupe $-NHSO_2CH_3$, un groupe

-$NHCOCH_2OH$, un groupe -$SO_2$-$CH_3$ ;

à l'état de base ou de sel d'addition à un acide.

Parmi les composés selon l'invention, on peut notamment citer les composés ci-après, tels quels ainsi que leurs sels :

| Nom IUPAC | Structure Chimique |
|---|---|
| 1<br>1'-{[4-(4-chlorophényl)-5-(2,4-dichlorophényl)-3-méthoxythién-2-yl]carbonyl}-1, 4'-bipip éridine-4'-carboxamide | 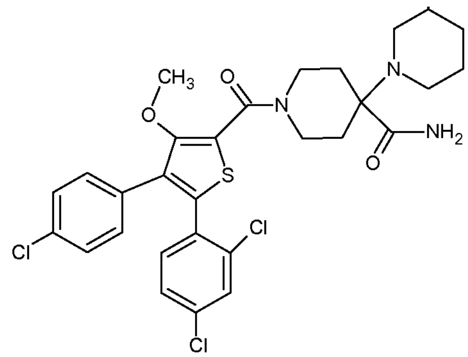 |
| 2<br>1'-{[4-(4-chlorophényl)-5-(2,4-dichlorophényl)-3-{3-[(méthylsulfonyl)amino]propoxy}thién-2-yl]carbonyl}-1, 4'-bipip éridine-4'-carboxamide | 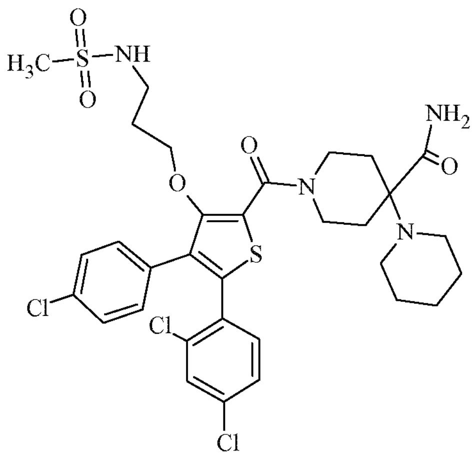 |
| 3<br>1'-({4-(4-chlorophényl)-5-(2,4-dichlorophényl)-3-[3-(méthylsulfonyl)propoxy]thién-2-yl}carbonyl)-1,4'-bipipéridine-4'-carboxamide | 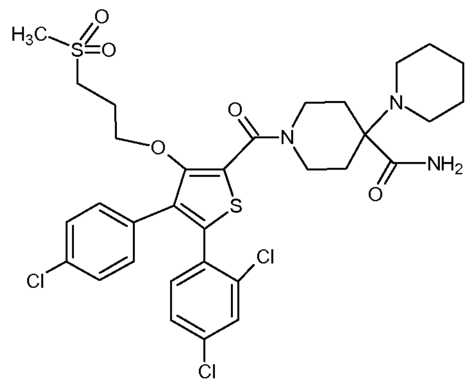 |

(suite)

| Nom IUPAC | Structure Chimique |
|---|---|
| 4<br>1-{[4-(4-chlorophényl)-5-(2,4-dichlorophényl)-3-(3-hydroxypropoxy)thién-2-yl]carbonyl}-4-phénylpipéridine-4-carboxamide | 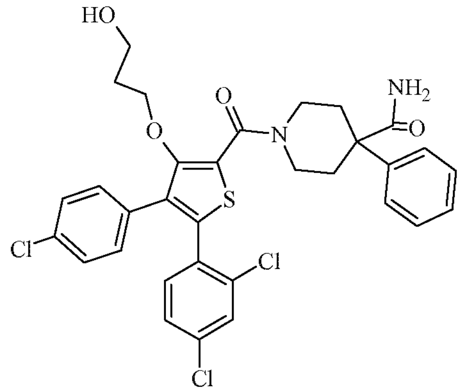 |
| 5<br>1-({4-(4-chlorophényl)-5-(2,4-dichlorophényl)-3-[3-(méthylsulfonyl)propoxy]thién-2-yl}carbonyl)-4-phénylpipéridine-4-carboxamide | 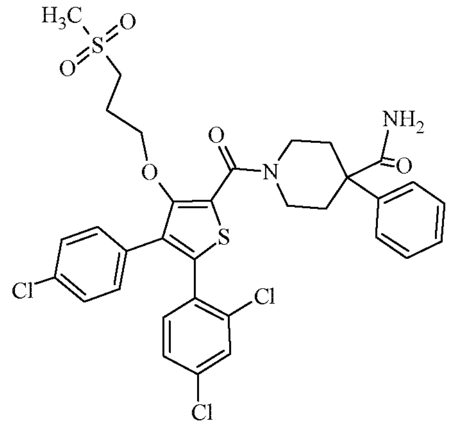 |
| 6<br>1-{[4-(4-chlorophényl)-5-(2,4-dichlorophényl)-3-(2-hydroxyéthoxy)thién-2-yl]carbonyl}-4-phénylpipéridine-4-carboxamide | 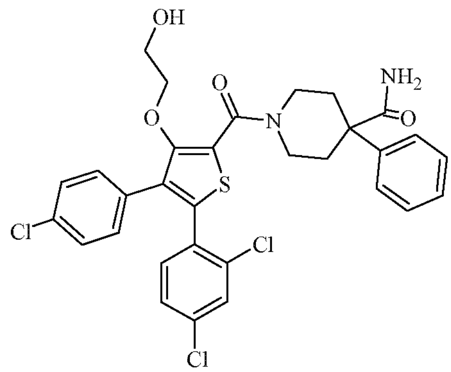 |
| 7<br>1'-{[4-(4-chlorophényl)-5-(2,4-dichlorophényl)-3-(2-hydroxyéthoxy)thién-2-yl]carbonyl}-4,4-difluoro-1,4'-bipipéridine-4'-carboxamide | 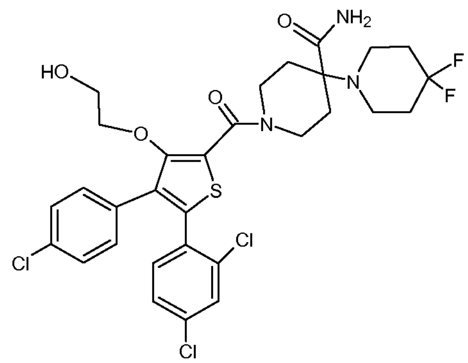 |

(suite)

| Nom IUPAC | Structure Chimique |
|---|---|
| 8<br>1'-({4-(4-chlorophényl)-5-(2,4-dichlorophényl)-3-[3-(méthylsulfonyl)propoxy]thién-2-yl}carbonyl)-4,4-difluoro-1,4'-bipipéridine-4'-carboxamide | 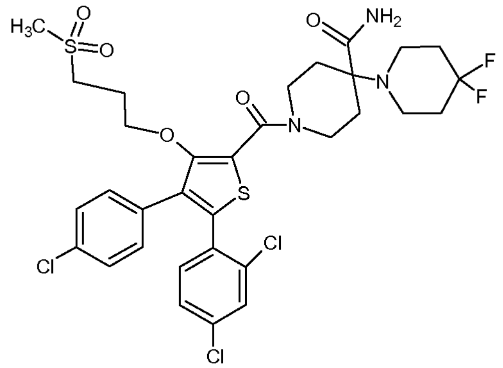 |
| 9<br>1'-{[4-(4-chlorophényl)-5-(2,4-dichlorophényl)-3-(3-hydroxypropoxy)thién-2-yl]carbonyl}-4,4-difluoro-1,4'-bipipéridine-4'-carboxamide | 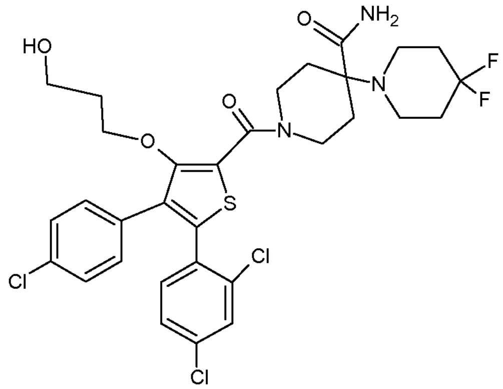 |
| 10<br>1-{[4-(4-chlorophényl)-5-(2,4-dichlorophényl)-3-(2-hydroxyéthoxy)thién-2-yl]carbonyl}-4-[(3,3,3-trifluoropropyl)amino]pipéridine-4-carboxamide | 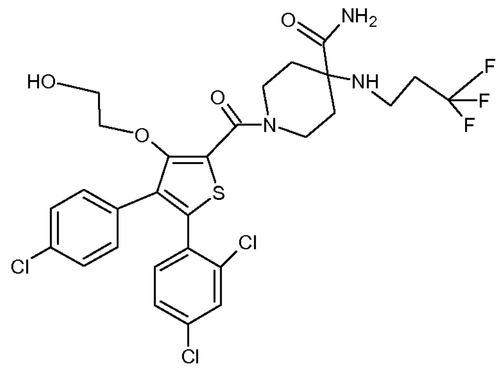 |
| 11<br>1-({4-(4-chlorophényl)-5-(2,4-dichlorophényl)-3-[3-(méthylsulfonyl)propoxy]thién-2-yl}carbonyl)-4-[(3,3,3-trifluoropropyl)amino]pipéridine-4-carboxamide | 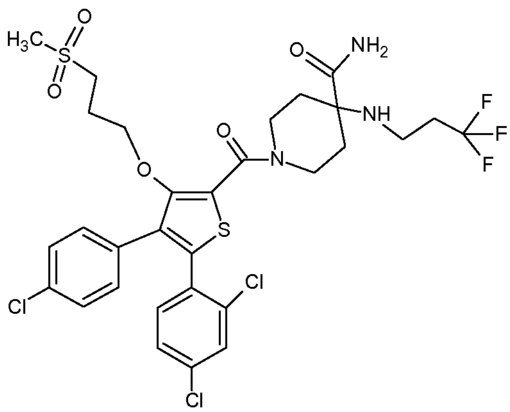 |

(suite)

| Nom IUPAC | Structure Chimique |
|---|---|
| 12<br>1-{[4-(4-chlorophényl)-5-(2,4-dichlorophényl)-3-{2-[(hydroxyacétyl)amino]éthoxy}thién-2-yl]carbonyl}-4-[(3,3,3-trifluoropropyl)amino]pipéridine-4-carboxamide | 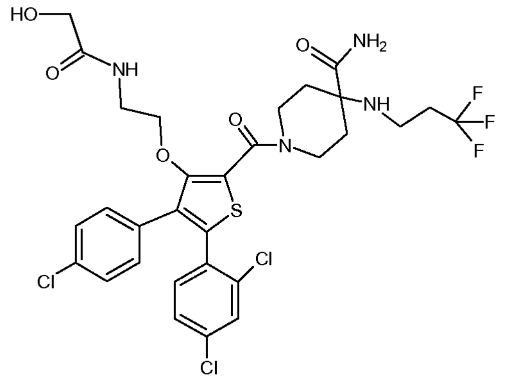 |
| 13<br>1-{[4-(4-chlorophényl)-5-(2,4-dichlorophényl)-3-(3-hydroxypropoxy)thién-2-yl]carbonyl}-4-[(3,3,3-trifluoropropyl)amino]pipéridine-4-carboxamide | 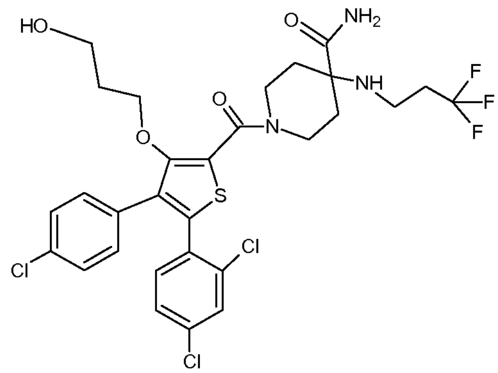 |
| 14<br>1-{[4-(4-chlorophényl)-5-(2,4-dichlorophényl)-3-{2-[(hydroxyacétyl)amino]éthoxy}thién-2-yl]carbonyl}-4-phénylpipéridine-4-carboxamide | 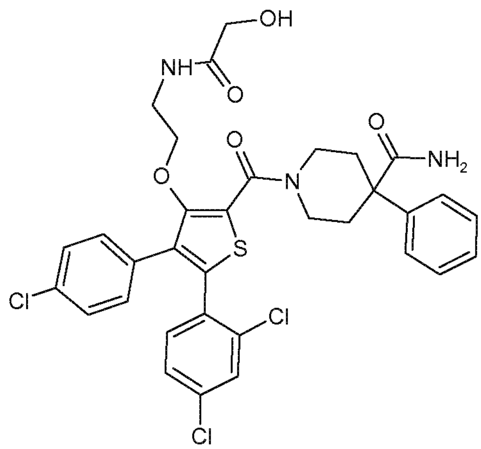 |
| 15<br>1-{[3-(2-acétamidoéthoxy)-4-(4-chlorophényl)-5-(2,4-dichlorophényl)thién-2-yl]carbonyl}-4-phénylpipéridine-4-carboxamide | 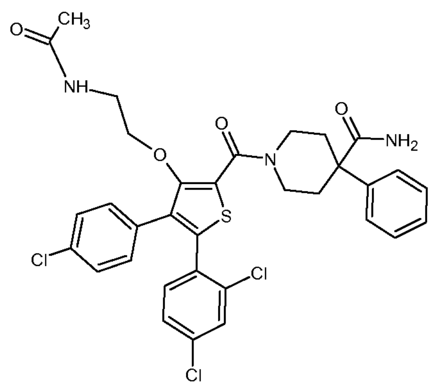 |

(suite)

| Nom IUPAC | Structure Chimique |
|---|---|
| 16<br>1-{[4-(4-chlorophényl)-5-(2,4-dichlorophényl)-3-(2-hydroxyéthoxy)thién-2-yl]carbonyl}-4-[(4-fluorobenzyl)amino]pipéridine-4-carboxamide | 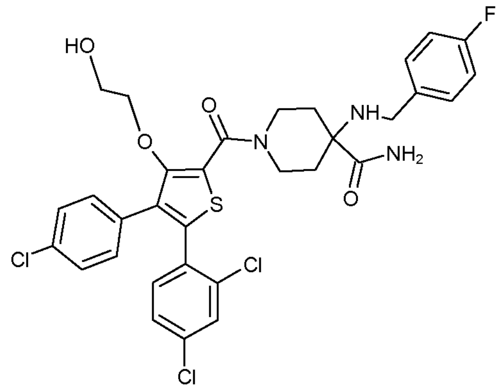 |
| 17<br>1-{[4-(4-chlorophényl)-5-(2,4-dichlorophényl)-3-(3-hydroxypropoxy)thién-2-yl]carbonyl}-4-[(4-fluorobenzyl)amino]pipéridine-4-carboxamide | 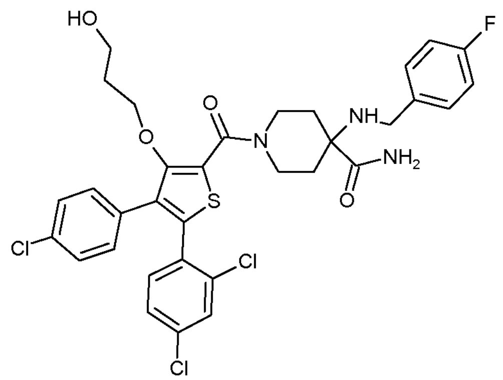 |
| 18<br>1-({4-(4-chlorophényl)-5-(2,4-dichlorophényl)-3-[3-(méthylsulfonyl)propoxy]thién-2-yl}carbonyl)-4-[(4-fluorobenzyl)amino]pipéridine-4-carboxamide | 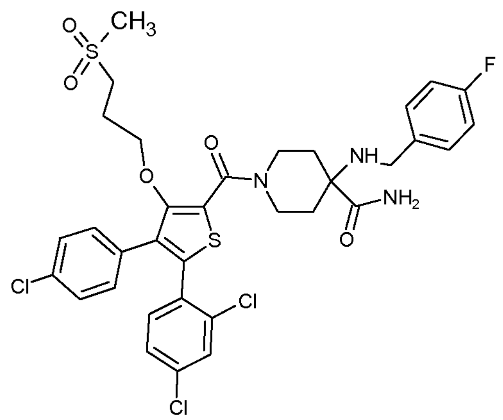 |
| 19<br>1-{[3-(2-acétamidoéthoxy)-4-(4-chlorophényl)-5-(2,4-dichlorophényl)thién-2-yl]carbonyl}-4-[(4-fluorobenzyl)amino]pipéridine-4-carboxamide | 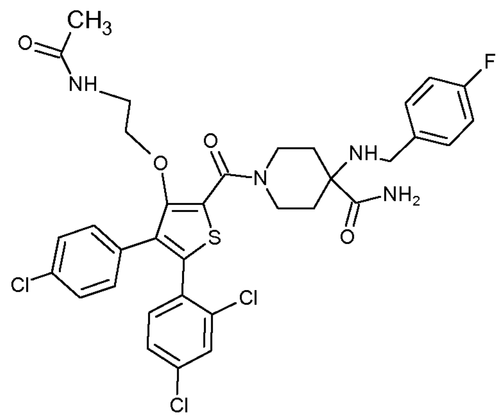 |

(suite)

| Nom IUPAC | Structure Chimique |
|---|---|
| 20<br>1-{[4-(4-chlorophényl)-5-(2,4-dichlorophényl)-3-{2-[(hydroxyacétyl)amino]éthoxy}thién-2-yl]carbonyl}-4-[(4-fluorobenzyl)amino]pipéridine-4-carboxamide | 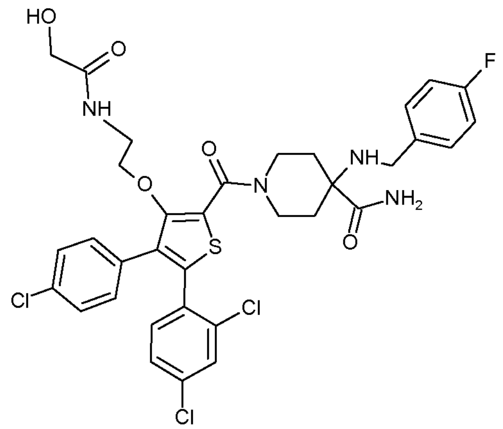 |
| 21<br>1-{[4-(4-chlorophényl)-5-(2,4-dichlorophényl)-3-{3-[(hydroxyacétyl)amino]propoxy}thién-2-yl]carbonyl}-4-[(4-fluorobenzyl)amino]pipéridine-4-carboxamide | 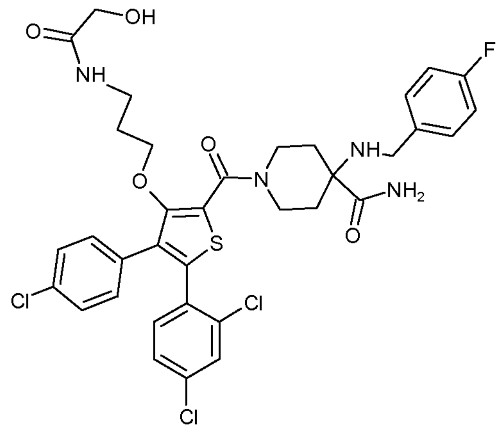 |
| 22<br>1-{[3-(3-acétamidopropoxy)-4-(4-chlorophényl)-5-(2,4-dichlorophényl)thién-2-yl]carbonyl}-4-[(4-fluorobenzyl)amino]pipéridine-4-carboxamide | 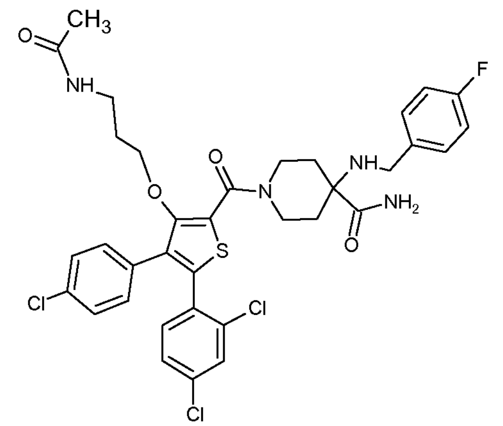 |
| 23<br>4-(benzylamino)-1-{[4-(4-chlorophényl)-5-(2,4-dichlorophényl)-3-(2-hydroxyéthoxy)thién-2-yl]carbonyl}pipéridine-4-carboxamide | 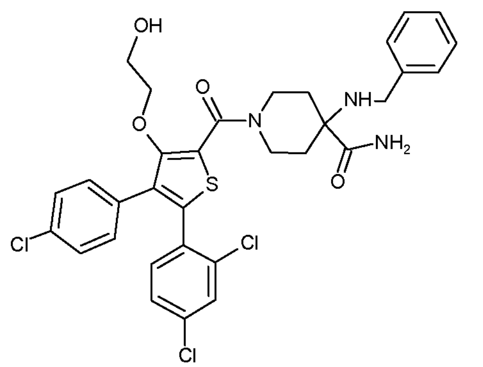 |

(suite)

| Nom IUPAC | Structure Chimique |
| --- | --- |
| 24<br><br>4-(benzylamino)-1-{[4-(4-chlorophényl)-5-(2,4-dichlorophényl)-3-(3-hydroxypropoxy)thién-2-yl]carbonyl}pipéridine-4-carboxamide | 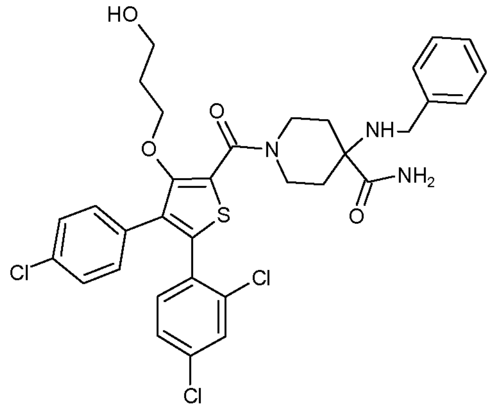 |
| 25<br><br>4-(benzylamino)-1-{[4-(4-chlorophényl)-5-(2,4-dichlorophényl)-3- {2-[(hydroxyacétyl)amino]éthoxy}thién-2-yl]carbonyl}pipéridine-4-carboxamide | 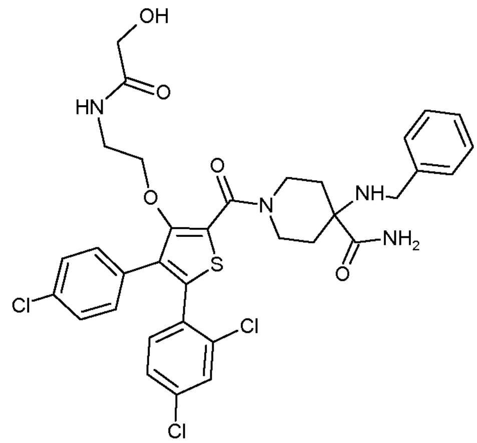 |
| 26<br><br>1-{[5-(2-chlorophényl)-4-(4-chlorophényl)-3-(3-hydroxypropoxy)thién-2-yl]carbonyl}-4-[(4-fluorobenzyl)amino]pipéridine-4-carboxamide | 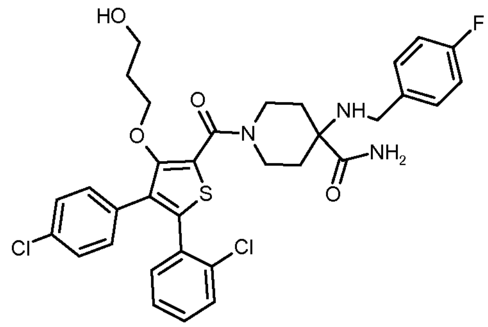 |
| 27<br><br>1-{[5-(2-chlorophényl)-4-(4-chlorophényl)-3-(3-hydroxypropoxy)thién-2-yl]carbonyl}-4-phénylpipéridine-4-carboxamide | 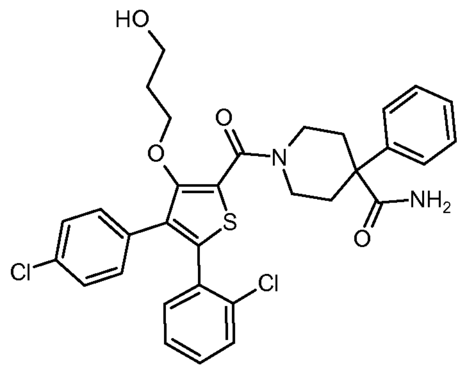 |

(suite)

| Nom IUPAC | Structure Chimique |
|---|---|
| 28<br>1-{[5-(2-chlorophényl)-4-(4-chlorophényl)-3-(2-hydroxyéthoxy)thién-2-yl]carbonyl}-4-phénylpipéridine-4-carboxamide | 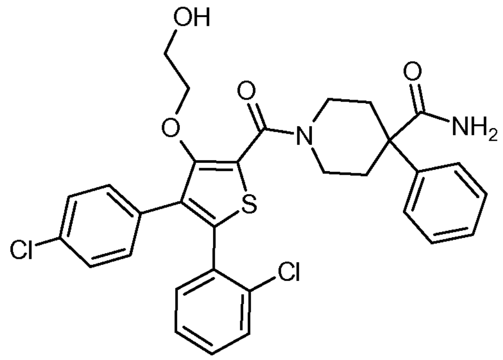 |
| 29<br>1-{[5-(2-chlorophényl)-4-(4-chlorophényl)-3-(2-hydroxyéthoxy)thién-2-yl]carbonyl}-4-[(4-fluorobenzyl)amino]pipéridine-4-carboxamide | 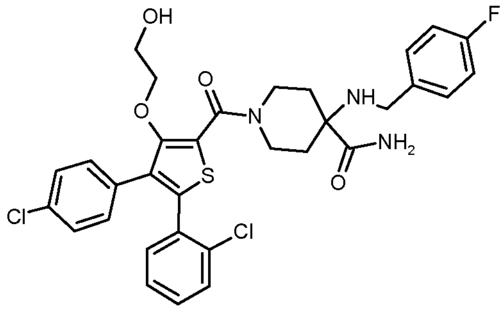 |
| 30<br>1-{[3-(2-aminoéthoxy)-5-(2-chlorophényl)-4-(4-chlorophényl)thién-2-yl]carbonyl}-4-[(4-fluorobenzyl)amino]pipéridine-4-carboxamide | 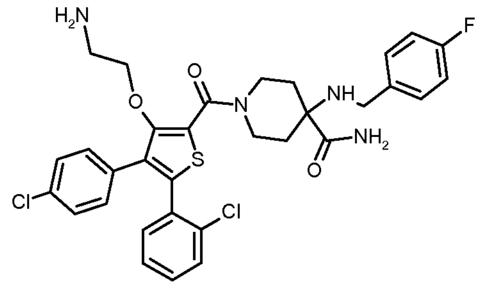 |
| 31<br>1-{[3-(2-acétamidoéthoxy)-5-(2-chlorophényl)-4-(4-chlorophényl)thién-2-yl]carbonyl}-4-[(4-fluorobenzyl)amino]pipéridine-4-carboxamide | 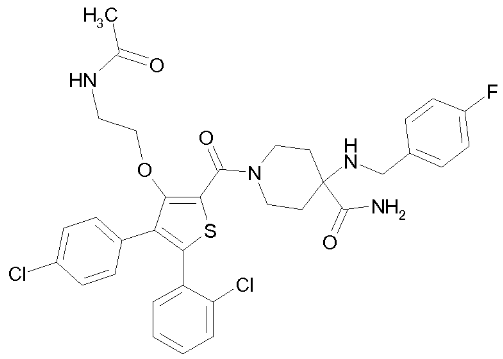 |

(suite)

| Nom IUPAC | Structure Chimique |
|---|---|
| 32<br>1-{[3-(2-aminoéthoxy)-5-(2-chlorophényl)-4-(4-chlorophényl)thién-2-yl]carbonyl}-4-phénylpipéridine-4-carboxamide | 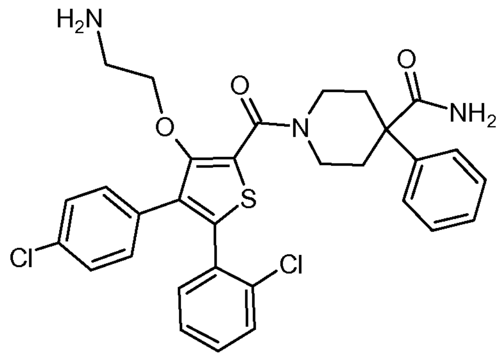 |
| 33<br>1-{[5-(2-chlorophényl)-4-(4-chlorophényl)-3-{2-[(hydroxyacétyl)amino]éthoxy}thién-2-yl]carbonyl}-4-phénylpipéridine-4-carboxamide | 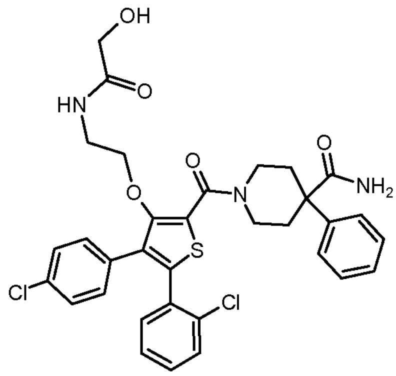 |
| 34<br>1-{[3-(2-acétamidoéthoxy)-5-(2-chlorophényl)-4-(4-chlorophényl)thién-2-yl]carbonyl}-4-phénylpipéridine-4-carboxamide | 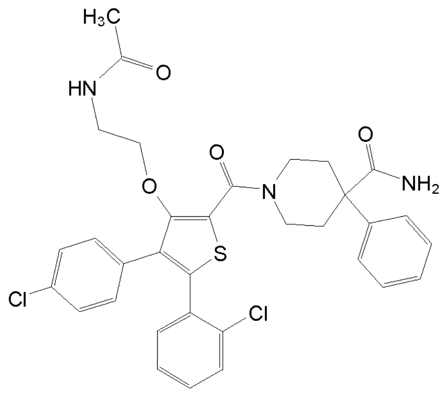 |
| 35<br>1-{[5-(2-chlorophényl)-4-(4-chlorophényl)-3-{2-[(hydroxyacétyl)amino]éthoxy}thién-2-yl]carbonyl}-4-[(4-fluorobenzyl)amino]pipéridine-4-carboxamide | 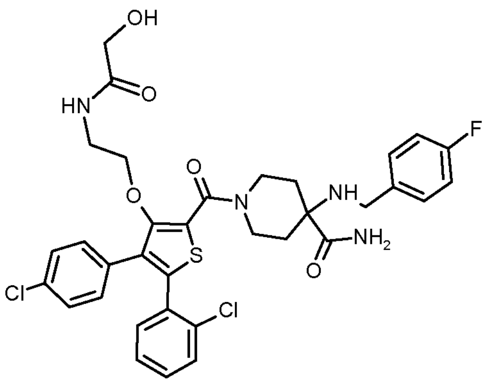 |

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont

données dans « Protective Group in Organic Synthesis », Green et al., 4th Edition, John Wiley & Sons, Inc., New York, 2007.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advanced Organic Chemistry », M. B. Smith and J. March, 6th Edition, Wiley Interscience, 2007, p.496-501.

Conformément à l'invention, on peut préparer les composés de formule (I) selon un procédé qui est caractérisé en ce que :

on fait réagir, en présence d'une base, un composé de formule :

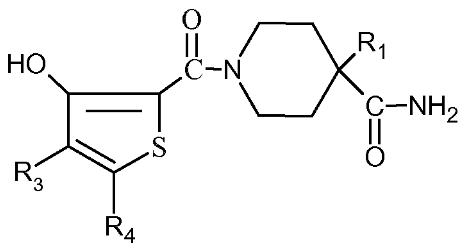

(II)

dans laquelle $R_1$, $R_3$ et $R_4$ sont tels que définis pour un composé de formule (I), avec un composé de formule :

$Z\text{-}R_2$ (III)

dans laquelle $R_2$ est tel que défini pour un composé de formule (I) et Z représente un groupe partant tel que précédemment décrit.

Eventuellement, on transforme le composé de formule (I) en l'un de ses sels d'addition à un acide.

La réaction s'effectue en présence d'une base telle qu'un carbonate de métal alcalin, le carbonate de césium ou le carbonate de sodium par exemple, dans un solvant tel que l'acétone, l'acétonitrile, le N,N-diméthylformamide et à une température comprise entre la température ambiante et la température de reflux du solvant.

Selon une variante du procédé :

On fait réagir un acide ou un dérivé fonctionnel de cet acide de formule :

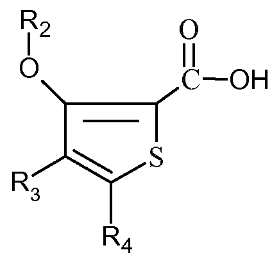

(IV)

dans laquelle $R_2$, $R_3$ et $R_4$ sont tels que définis pour un composé de formule (I), avec un composé de formule :

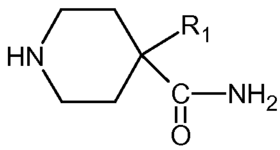

(V)

dans laquelle $R_1$ est tel que défini pour un composé de formule (I).

Eventuellement, on transforme le composé de formule (I) ainsi obtenu en un de ses sels.

Lorsqu'on traite un composé de formule (V) avec l'acide de formule (IV) lui-même, on opère en présence d'un agent de couplage utilisé en chimie peptidique tel que le 1,3-dicyclohexylcarbodiimide ou l'hexafluorophosphate de benzotriazol-1-yl-oxytris(diméthylamino) phosphonium ou l'hexafluorophosphate de benzotriazol-1-yl-oxytris(pyrrolidino) phosphonium ou le tétrafluoroborate de 2-(1*H*-benzotriazol-1-yl)-1,1,3,3-tétraméthyl uronium, en présence d'une base telle que la triéthylamine, la N,N-diisopropyléthylamine ou la 4-diméthylaminopyridine, dans un solvant tel que le dichlorométhane, le dichloroéthane, le N-N-diméthylformamide, le tétrahydrofurane à une température comprise entre -10°C et la température de reflux du solvant.

Comme dérivé fonctionnel de l'acide (IV) on peut utiliser le chlorure d'acide, l'anhydride, un anhydride mixte, un ester alkylique en $C_1$-$C_4$ dans lequel l'alkyle est droit ou ramifié, un ester activé, par exemple l'ester de *p*-nitrophényle.

Ainsi dans le procédé selon l'invention, on peut aussi faire réagir le chlorure de l'acide obtenu par réaction du chlorure de thionyle ou du chlorure d'oxalyle sur l'acide de formule (IV), avec le composé de formule (V), dans un solvant, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préparer l'anhydride mixte de l'acide de formule (IV) par réaction du chloroformiate d'éthyle avec l'acide de formule (IV), en présence d'une base telle que la triéthylamine, et à le faire réagir avec le composé de formule (V), dans un solvant tel que le dichlorométhane, sous une atmosphère inerte, à la température ambiante, en présence d'une base telle que la triéthylamine.

Selon une autre variante du procédé, on peut préparer les composés de formule (I) dans laquelle $R_2$ représente un groupe -X-$R_7$ et $R_7$ représente un groupe -$NR_9R_{10}$ avec $R_{10}$ = -$COR_{11}$ ou avec $R_{10}$ = -$CO(CH_2)_mOH$ à partir d'un composé de formule (I) dans laquelle X-$NR_9R_{10}$ = X-$NR_9$H. Ce dernier composé est mis en réaction avec un acide de formule $R_{11}$-COOH (VI) ou, respectivement, avec un acide de formule HO-$(CH_2)_m$-COOH (VII) selon les conditions opératoires précédemment décrites pour la réaction d'un acide de formule (IV) avec le composé de formule (V). Selon une autre variante du procédé, on peut préparer les composés de formule (I) dans laquelle $R_2$ représente un groupe -X-$R_7$ et $R_7$ représente un groupe -$NR_9R_{10}$ avec $R_{10}$ = -$SO_2R_{11}$ à partir d'un composé de formule (I) dans laquelle X-$NR_9R_{10}$ = X-$NR_9$H. Ce dernier composé est mis en réaction avec un composé de formule Hal-$SO_2R_{11}$ (VIII) dans laquelle Hal représente un atome d'halogène de préférence le chlore. La réaction s'effectue en présence d'une base telle que la triéthylamine dans un solvant tel que le dichlorométhane et à une température comprise entre 0°C et la température ambiante.

Les composés de formule (I) obtenus par les différents modes opératoires peuvent être ultérieurement séparés du milieu réactionnel et purifiés selon les méthodes classiques, par exemple par cristallisation ou chromatographie.

Les composés de formule (II) se préparent par réaction d'un composé de formule :

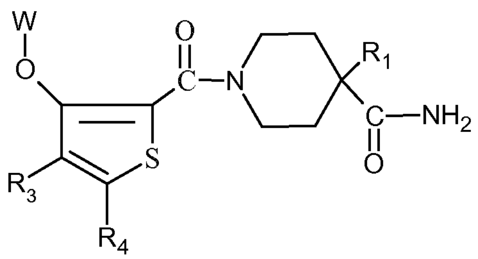

(IX)

dans laquelle $R_1$, $R_3$ et $R_4$ sont tels que définis pour un composé de formule (I) et W représente un ($C_1$-$C_4$)alkyle, avec, par exemple, du tribromoborane, dans un solvant tel que, par exemple, le dichlorométhane et à une température comprise entre -60°C et la température ambiante.

Les composés de formule (III) sont connus, commercialisés ou se préparent selon des méthodes connues de l'homme de l'art.

Les composés de formule (IV) se préparent par saponification d'un composé de formule :

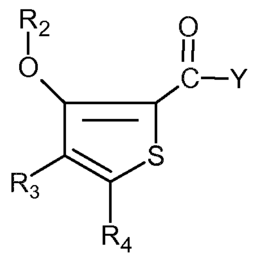

(X)

dans laquelle $R_2$, $R_3$ et $R_4$ sont tels que définis pour un composé de formule (I) et Y représente un groupe ($C_1$-$C_2$)alcoxy. Ainsi, par exemple, lorsque Y = $-OCH_3$, la réaction s'effectue par action de la soude, dans un solvant tel que le méthanol, le N,N-diméthylformamide ou un mélange de ces solvants et à une température comprise entre la température ambiante et la température de reflux du solvant.

Les composés de formule (V) sont connus ou se préparent selon des méthodes connues telles que celles décritent dans EP 0 428 434, EP 0 512 901, EP 0 515 240, WO 96/23 787, WO 02/094 821, WO 03/104 225 et WO 2006/021 654 ou dans J. Med. Chem., 1964, 7, 619-622 et J. Org. Chem., 1990, 55, 4207-4209.

Les composés de formule (V) sont généralement préparés sous forme protégée sur l'atome d'azote de la pipéridine ; après une étape de déprotection, on obtient les composés de formule (V) attendus.

Les composés de formule (VI), (VII) et (VIII) sont connus, commercialisés ou se préparent selon des méthodes connues de l'homme de l'art.

Les composés de formule (IX) se préparent par réaction d'un acide ou d'un dérivé fonctionnel de cet acide de formule :

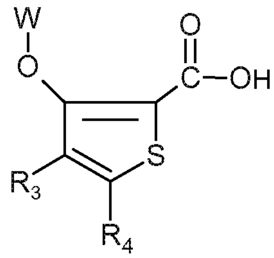

(XI)

dans laquelle $R_3$ et $R_4$ sont tels que définis pour un composé de formule (I) et W représente un ($C_1$-$C_4$)alkyle, avec un composé de formule (V) selon les conditions opératoires précédemment décrites pour la réaction d'un composé de formule (IV) avec le composé de formule (V).

Les composés de formule (X) se préparent par réaction d'un composé de formule :

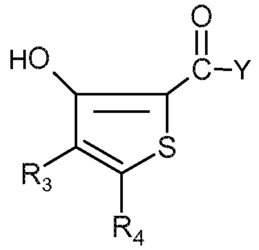

(XII)

dans laquelle $R_3$ et $R_4$ sont tels que définis pour un composé de formule (I) et Y représente un ($C_1$-$C_2$)alcoxy, avec un composé de formule (III) selon les conditions opératoires précédemment décrites pour la réaction d'un composé de formule (II) avec le composé de formule (III).

Les composés de formule (XI) se préparent par saponification d'un composé de formule :

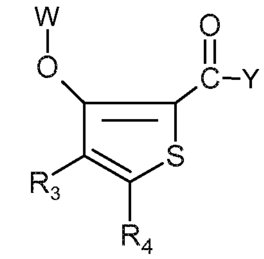

(XIII)

dans laquelle $R_3$ et $R_4$ sont tels que définis pour un composé de formule (I), W représente un ($C_1$-$C_4$)alkyle et Y représente un ($C_1$-$C_2$)alcoxy, selon les méthodes précédemment décrites pour la saponification d'un composé de formule (X).

Les composés de formule (XII) se préparent par réaction d'un composé de formule (XIII) avec, par exemple, du tribromoborane selon les conditions opératoires précédemment décrites pour la préparation d'un composé de formule (II).

Les composés de formule (XIII) se préparent selon le SCHEMA I ci-après dans lequel Hal représente un atome d'halogène, de préférence le brome, Y représente un ($C_1$-$C_2$)alcoxy et W représente un ($C_1$-$C_4$)alkyle, de préférence un méthyle.

SCHEMA I

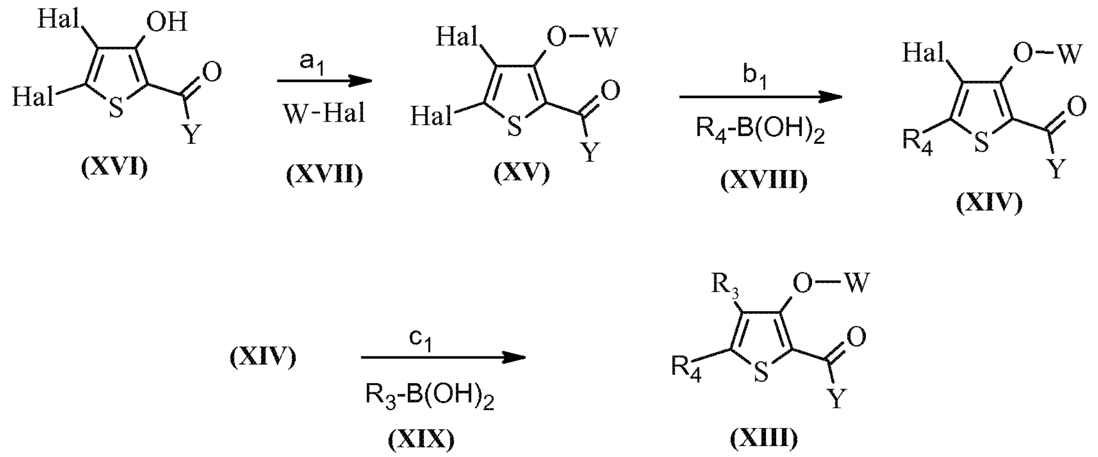


(XVI) (XVII) (XV) (XVIII) (XIV)

(XIV) (XIX) (XIII)

A l'étape al du Schéma I, on fait réagir un composé de formule (XVI) avec un composé de formule W-Hal (XVII) en présence d'une base telle qu'un carbonate de métal alcalin, le carbonate de potassium par exemple, dans un solvant tel que le N,N-diméthylformamide et à une température comprise entre la température ambiante et la température de reflux du solvant.

A l'étape b1, le composé de formule (XV) ainsi obtenu est mis en réaction avec un acide boronique de formule $R_4$-$B(OH)_2$ (XVIII) dans un milieu basique par exemple en présence d'un carbonate de métal alcalin tel que le carbonate de sodium par exemple, et en présence d'un complexe du palladium tel que, par exemple, le tétrakis (triphénylphosphine)palladium, dans un solvant tel que le toluène, le tétrahydrofurane ou le dioxane et à une température comprise entre la température ambiante et la température de reflux du solvant.

A l'étape c1, le composé de formule (XIV) ainsi obtenu est mis en réaction avec un acide boronique de formule $R_3$-$B(OH)_2$ (XIX) selon les conditions précédemment décrites à l'étape b1.

Les composés de formule (XVI), (XVII), (XVIII) et (XIX) sont connus, commercialisés ou se préparent selon des méthodes connues de l'homme de l'art.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans les TABLEAUX (I) et (II) ci-après, qui illustrent les structures chimiques et les propriétés physiques de quelques composés selon l'invention.

Dans les Préparations et dans les Exemples on utilise les abréviations suivantes :

AcOEt : acétate d'éthyle
$BBr_3$ : tribromure de bore
$Cs_2CO_3$ : carbonate de césium

DCM : dichlorométhane
DMSO : diméthylsulfoxyde
DMF : N,N-diméthylformamide
Eb : température d'ébullition
éther : éther diéthylique
éther chlorhydrique 2N : solution 2N d'acide chlorhydrique dans l'éther diéthylique
éther iso : éther diisopropylique
F : point de fusion
HPLC : chromatographie en phase liquide sous haute pression
UPLC : chromatographie en phase liquide « ultraperformance »
MeOH : méthanol
Silice H : gel de silice 60 H commercialisé par Merck (DARMSTAD)
Solution tampon pH = 2 : solution de 16,66 g de $KHSO_4$ et 32,32 g de $K_2SO_4$ dans 1 litre d'eau.
TA : température ambiante
TBTU : O-benzotriazol-1-yl-N, N, N', N'- tétraméthyluronium tetrafluoroborate
TFA : acide trifluoroacétique
THF : tétrahydrofurane
Tetrakis : tétrakis(triphénylphosphine)palladium

Les spectres de résonance magnétique nucléaire sont enregistrés dans le DMSO-d6. Pour l'interprétation des spectres, on utilise les abréviations suivantes : s : singulet, d : doublet, t : triplet, q : quadruplet, qui : quintuplet, m : massif, sl : singulet large, dd : doublet dedoublé.

Les composés selon l'invention sont analysés par couplage LC/UV/MS (chromatographie liquide/détection UV/spectrométrie de masse). On mesure le pic moléculaire caractéristique ($MH^+$) et le temps de rétention (tr) en minutes (min).

Les composés sont analysés par couplage HPLC-UV-MS ou bien UPLC-UV-MS (chromatographie liquide-détection UV et détection masse).

Les conditions analytiques sont les suivantes :

Conditions A (HPLC) :

On utilise une colonne: Symmetry C18 (50 x 2,1 mm ; 3,5 μm)
Eluant A : 0,005 % de TFA dans l'eau à environ pH 3,1
Eluant B : 0,005% de TFA 0.005% dans l'acétonitrile.
Gradient:

| Temps (min) | % A | % B |
|---|---|---|
| 0 | 100 | 0 |
| 10 | 10 | 90 |
| 15 | 10 | 90 |
| 16 | 100 | 0 |
| 20 | 100 | 0 |

Température colonne : 30°C ; débit : 0,4 ml/minute.
Détection : $\lambda$ = 210 nm - 220 nm

Conditions B (HPLC) :

On utilise une colonne XTerra MS C18 (50 x 2,1 mm ; 3,5 μm)
Eluant A : $AcONH_4$ 10mM à environ pH 7
Eluant B : acétonitrile
Gradient :

| Temps (min) | % A | % B |
|---|---|---|
| 0 | 100 | 0 |
| 10 | 10 | 90 |
| 15 | 10 | 90 |
| 16 | 100 | 0 |
| 20 | 100 | 0 |

Température colonne : 30°C ; débit : 0,4 ml/minute.
Détection : λ = 220 nm

Conditions C (UPLC) :

On utilise une colonne Acquity BEH C18 (50 x 2,1 mm ; 1,7 μm)
Eluant A : 0,005 % de TFA dans l'eau à environ pH 3,1 / acétonitrile (97/3)
Eluant B : 0,035% de TFA dans l'acétonitrile.
Gradient:

| Temps (min) | % A | % B |
|---|---|---|
| 0 | 100 | 0 |
| 2.3 | 5 | 95 |
| 2.9 | 5 | 95 |
| 3 | 100 | 0 |
| 3.5 | 100 | 0 |

Température colonne : 40°C ; débit : 1 ml/minute.
Détection : λ = 220 nm

Conditions de spectrométrie de masse

L'enregistrement des spectres de masse est effectué en mode electrospray (ESI) positif, afin d'observer les ions issus de la protonation de composés analysés ($MH^+$), ou de la formation d'adduits avec d'autres cations tels que $Na^+$, $K^+$, etc.

## PREPARATIONS

**1- Préparation de composés de formule (XIII)**

Préparation 1.1 :

4-(4-Chlorophényl)-5-(2,4-dichlorophényl)-3-méthoxythiophène-2-carboxylate de méthyle.

(XIII) : $R_3$ = 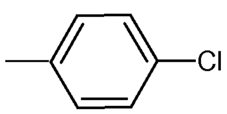 ; $R_4$ = 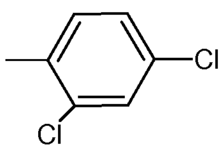 ; W = $-CH_3$ ; Y = $-OCH_3$

A) 4,5-Dibromo-3-méthoxythiophène-2-carboxylate de méthyle.

A une solution de 34,35 g de 4,5-dibromo-3-hydroxythiophène-2-carboxylate de méthyle dans 150 ml de DMF,

on ajoute, goutte à goutte, 9,97 ml d'une solution d'iodométhane puis 29,44 g de $K_2CO_3$ et laisse sous agitation pendant 18 heures à TA. Après refroidissement, on dilue le mélange réactionnel dans 100 ml d'éther éthylique, filtre et concentre le filtrat sous vide. On purifie le brut réactionnel par chromatographie sur gel de silice en éluant à l'heptane puis par un mélange heptane/AcOEt (85/15; v/v). On obtient 28,70 g du composé attendu.

B) 4-Bromo-5-(2,4-dichlorophényl)-3-méthoxythiophène-2-carboxylate de méthyle

A une solution de 28,70 g du composé de l'étape A) dans 110 ml de toluène, on ajoute 16,60 g d'acide (2,4-dichlorophényl)boronique puis, goutte à goutte, 87 ml d'une solution 2M de $Na_2CO_3$. On fait barboter de l'argon dans le mélange réactionnel pendant 30 minutes, ajoute ensuite 5,03 g de Tétrakis et chauffe à 110°C pendant 18 heures. Après refroidissement à TA, on ajoute 80 ml d'eau distillée dans le mélange réactionnel, extrait avec 80 ml d'AcOEt, sèche sur $Na_2SO_4$ et évapore le solvant sous vide. On purifie le brut réactionnel par chromatographie sur gel de silice en éluant à l'heptane puis par un mélange heptane/AcOEt (85/15; v/v). On obtient 16,21 g du composé attendu.

C) 4-(4-Chlorophényl)-5-(2,4-dichlorophényl)-3-méthoxythiophène-2-carboxylate de méthyle

A une solution de 16,21 g du composé de l'étape B) dans 110 ml de toluène, on ajoute 7,75 g d'acide (4-chlorophényl)boronique puis, goutte à goutte, 40,93 ml d'une solution 2M de $Na_2CO_3$. On fait barboter de l'argon dans le mélange réactionnel pendant 30 minutes, ajoute ensuite 2,36 g de Tétrakis et chauffe à 110°C pendant 18 heures. Après refroidissement à TA, on rince le mélange réactionnel avec 80 ml d'eau distillée puis extrait avec 80 ml d'AcOEt, sèche sur $Na_2SO_4$ évapore le solvant sous vide. On purifie le brut réactionnel par chromatographie sur gel de silice en éluant à l'heptane puis par un mélange heptane/AcOEt (85/15; v/v). On obtient 13,23 g du composé attendu.

Préparation 1.2 :

5-(2-Chlorophényl)-4-(4-chlorophényl)-3-méthoxythiophène-2-carboxylate de méthyle.

(XIII) : $R_3$ = 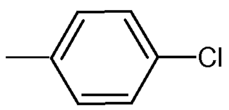 ; $R_4$ = 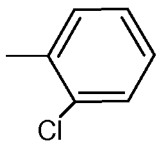 ; W = $-CH_3$ ; Y = $-OCH_3$

A) 4-Bromo-5-(2-chlorophenyl)-3-methoxy-thiophene-2-carboxylate de méthyle.

Dans 60 ml de toluène on ajoute 11,39 g de composé de l'étape A de la Préparation 1.1, 5,56 g d'acide 2-chlorophénylboronique et 34,52 ml d'une solution 2M de carbonate de sodium. On fait buller de l'argon durant 30 min avant d'ajouter 1,99 g de Tetrakis. On chauffe à 110°C pendant la nuit. De retour à température ambiante on ajoute 60 ml d'eau distillée et extrait avec 60 ml d'acétate d'éthyle, on sèche et évapore la phase organique. Le produit obtenu est purifié par chromatographie sur silice (éluant : Heptane/Acétate d'éthyle (80/20 ; v/v)). On obtient 7,84 g du composé attendu.

B) 5-(2-Chlorophenyl)-4-(4-chlorophenyl)-3-methoxy-thiophene-2-carboxylate de méthyle.

Dans 60 ml de toluène on ajoute 7,84 g de composé de l'étape A, 4,10 g d'acide 4-chlorophénylboronique, 21,68 ml d'une solution 2M de carbonate de sodium. On fait buller de l'argon durant 30 min avant d'ajouter 1,25 g de Tetrakis. On chauffe à 110°C pendant la nuit. De retour à température ambiante on ajoute 60 ml d'eau distillée et extrait avec 60 ml d'acétate d'éthyle, on sèche et évapore la phase organique. Le produit obtenu est purifié par chromatographie sur silice (éluant : Heptane/Acétate d'éthyle (80/20 ; v/v)). On obtient 4,90 g du composé attendu.

**2- Préparation de composés de formule (XI)**

Préparation 2.1 :

Acide 4-(4-chlorophényl)-5-(2,4-dichlorophényl)-3-méthoxythiophène-2-carboxylique.

(XI) : 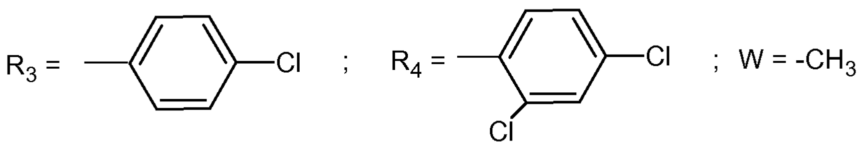

A un mélange de 13,23 g du composé de la préparation 1.1 dans un mélange de 60 ml DMF/MeOH (50/50 ; v/v), on ajoute 1,86 g de NaOH en pastilles et on chauffe à 80°C pendant 18 heures. On évapore à sec, reprend le résidu avec 30 ml d'eau distillée, lave la phase aqueuse avec 50 ml d'éther éthylique. On acidifie ensuite la phase aqueuse par ajout d'HCl jusqu'à pH = 2, essore et sèche. On obtient 12,43 g du composé attendu.

**3- Préparation de composés de formule (IX)**

Préparation 3.1 :

1-{[4-(4-Chlorophényl)-5-(2,4-dichlorophényl)-3-méthoxythién-2-yl]carbonyl}-4-phénylpipéridine-4-carboxamide.

(IX) : 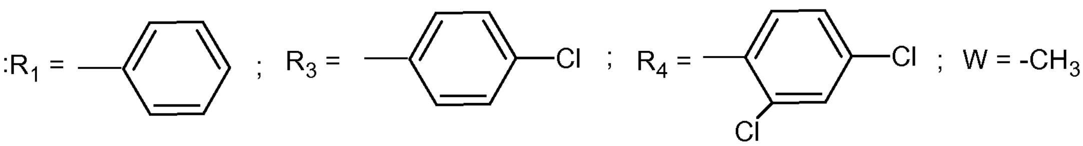

A un mélange de 2 g du composé de la Préparation 2.1 dans 100 ml de DCM, on ajoute 1,16 g de chlorhydrate de 4-phénylpipéridine-4-carboxamide, puis goutte à goutte, 2,02 ml d'une solution de triéthylamine et 1,71 g de TBTU. On laisse sous agitation pendant 1 heure à température ambiante. On évapore le solvant sous vide, ajoute une solution tampon pH = 2, extrait à l'AcOEt, sèche, filtre et concentre. On purifie le brut réactionnel par chromatographie sur gel de silice en éluant au DCM puis par le mélange DCM/MeOH (90/10 ; v/v). On obtient 1,96 g du composé attendu.

Préparation 3.2 :

4-(Benzylamino)-1-[[4-(4-chlorophényl)-5-(2,4-dichlorophényl)-3-méthoxy-2-thiényl]carbonyl]pipéridine-4-carboxamide.

(IX) : 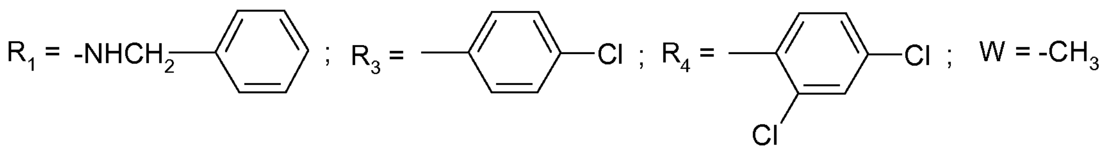

A un mélange de 1,64 g du composé de la Préparation 2.1 dans 50 ml de DCM, on ajoute 1,34 g de dichlorhydrate de 4-(benzylamino)pipéridine-4-carboxamide, 1,93 ml de triéthylamine et 1,4 g de TBTU et laisse 1 heure sous agitation à température ambiante. On concentre le mélange réactionnel sous vide, reprend le résidu par une solution tampon pH = 2, extrait à l'AcOEt, sèche la phase organique sur $MgSO_4$, filtre et évapore le solvant sous vide. On chromatographie le résidu sur gel de silice en éluant au DCM puis par le mélange DCM/MeOH (60/40 ; v/v). On obtient 2,0 g du composé attendu.

**4- Préparation de composés de formule (II)**

Préparation 4.1 :

1-{[4-(4-Chlorophényl)-5-(2,4-dichlorophényl)-3-hydroxythién-2-yl]carbonyl}-4-phénylpipéridine-4-carboxamide.

(II) : $R_1$ = 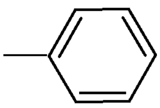 ; $R_3$ = 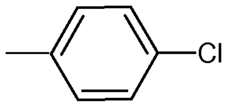 ; $R_4$ = 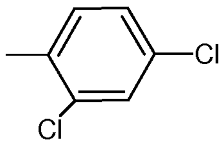

On refroidit à -50°C un mélange de 1,81 g du composé de la Préparation 3.1 dans 20 ml de DCM, puis ajoute, goutte à goutte, 9,05 ml d'une solution 1 M de tribromoborane et laisse remonter la température à TA sous agitation pendant 4 heures. On verse le mélange réactionnel sur de la glace, neutralise à pH = 7 par ajout de NaOH concentrée, extrait au DCM, sèche et concentre. On purifie le brut réactionnel par chromatographie sur gel de silice en éluant au DCM puis par le mélange DCM/MeOH (97/3 ; v/v). On obtient 0,715 g du composé attendu.

Préparation 4.2 :

1-{[4-(4-Chlorophényl)-5-(2,4-dichlorophényl)-3-hydroxythién-2-yl]carbonyl}-4-cyclohexylpipéridine-4-carboxamide.

(II) : $R_1$ = 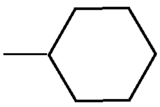 ; $R_3$ = 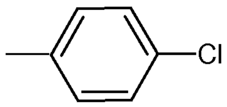 ; $R_4$ = 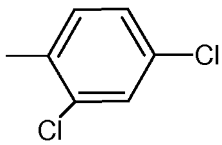

On prépare ce composé en suivant les modes opératoires décrits aux Préparations 3.1 et 4.1 à partir de la 4-cyclohexylpipéridine-4-carboxamide et du composé de la Préparation 2.1.

Préparation 4.3 :

4-(Benzylamino)-1-[[4-(4-chlorophényl)-5-(2,4-dichlorophényl)-3-hydroxy-2-thiényl]carbonyl]pipéridine-4-carboxamide.

(II) : $R_1$ = $-NHCH_2$ 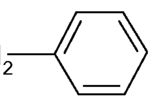 ; $R_3$ = 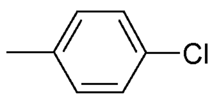 ; $R_4$ = 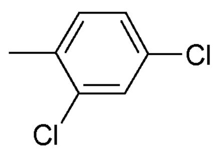

On refroidit à -50°C un mélange de 2,0 g du composé de la Préparation 3.2 dans 40 ml de DCM, puis ajoute, goutte à goutte, 9,54 ml d'une solution 1 M de tribromoborane dans du DCM et laisse 4 heures sous agitation en laissant remonter la température à TA. On verse le mélange réactionnel sur de la glace, neutralise à pH = 7 par ajout de NaOH concentrée, extrait au DCM, sèche la phase organique sous $MgSO_4$, filtre et évapore le solvant sous vide. On reprend le résidu dans l'éther iso, essore le précipité formé et le sèche sous vide. On obtient 1,92 g du composé attendu.

**5- Préparation de composés de formule (XII)**

Préparation 5.1 :

5-(2-Chlorophényl)-4-(4-chlorophényl)-3-hydroxythiophène-2-carboxylate de méthyle.

(XII) : $R_3$ = 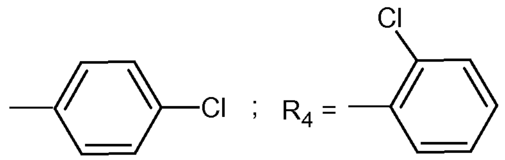 ; $R_4$ = ; Y = $OCH_3$

Dans 20 ml de DCM on ajoute 3,35 g du composé de la Préparation 1.2 puis on refroidit à -50°C, ajoute goutte à goutte 6,40 ml de BBr3 (1M dans le DCM) puis on laisse remonter à température ambiante. On concentre, reprend avec du DCM, lave avec 50 ml d'eau, sèche et concentre. Le produit obtenu est purifié par chromatographie sur silice (éluant : Heptane/Acétate d'éthyle (80/20 ; v/v)). On obtient 2,47 g du composé attendu.

**6- Préparation de composés de formule (X)**

Préparation 6.1 :

5-(2-Chlorophényl)-4-(4-chlorophényl)-3-[3-(tétrahydro-2*H*-pyran-2-yloxy)-propoxy]-thiophène-2-carboxylate de méthyle.

(X) : $R_2$ = $-(CH_2)_3-O$ 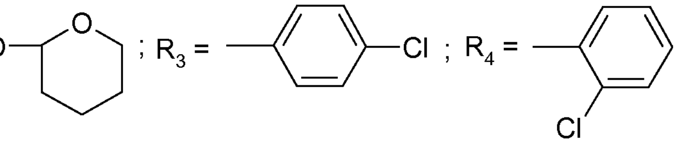 ; $R_3$ = ; $R_4$ = ; Y = $OCH_3$-

Dans 20 ml d'acétone on ajoute 0,7 g du composé de la Préparation 5.1, 0,824 g de 2-(3-bromo-propoxy)-tétrahydro-2H-pyrane et 1,2 g de $Cs_2CO_3$ et chauffe à reflux durant la nuit. On filtre et concentre. Le produit obtenu est purifié par chromatographie sur silice (éluant : Heptane/Acétate d'éthyle (60/40 ; v/v)). On obtient 0,950 g du composé attendu.

**7- Préparations de composés de formule (IV)**

Préparation 7.1 :

Acide 5-(2-chlorophényl)-4-(4-chlorophényl)-3-[3-(tétrahydro-2*H*-pyran-2-yloxy)-propoxy]-thiophène-2-carboxylique.

(IV) : $R_2$ = $-(CH_2)_3-O$ 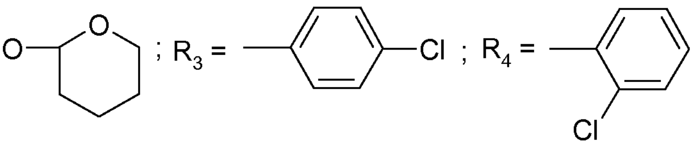 ; $R_3$ = ; $R_4$ =

On chauffe à reflux pendant 3 heures un mélange de 0,95 g du composé de la Préparation 6.1, et 0,29 g de NaOH en pastilles dans 40 ml de MeOH et 1 ml d'eau. On concentre le mélange réactionnel sous vide, reprend le résidu dans une solution tampon PH = 2, extrait au DCM, sèche la phase organique sur $MgSO_4$, filtre et évapore le solvant sous vide. On chromatographie le résidu sur le gel de silice en éluant au DCM puis par le gradient du mélange DCM/MeOH

jusqu'à (80/40 ; v/v)). On obtient 0,564 g du composé attendu.

**8- Préparation de composés de formule (V)**

Préparation 8.1 :

4-[(3,3,3-Trifluoropropyl)amino]-4-pipéridinecarboxamide.

(V) : $R_1$ = $-NH(CH_2)_2-CF_3$

A) 4-Carbamoyl-4-[(3,3,3-trifluoropropyl)amino]pipéridine-1-carboxylate de *tert*-butyle.

Dans 250 ml de DCM on ajoute à TA, 10 g de 4-amino-4-carbamoylpipéridine-1-carboxylate de tert-butyle, 4,42 g de 3,3,3-trifluoro-propionaldéhyde, 17,61 g de triacétoxyborohydrure de sodium, 4,51 ml d'acide acétique puis on agite le mélange pendant 3 heures. On ajoute de l'eau et extrait le composé attendu au DCM, lave la phase organique avec une solution saturé de $NaHCO_3$, puis avec de l'eau. Après séchage de la phase organique, filtration et évaporation à sec, on obtient 13 g de composé attendu.

B) 4-[(3,3,3-trifluoropropyl)amino]pipéridine-4-carboxamide.

On agite 13 g de composé obtenu en (A) dans 25 ml de MeOH, puis on ajoute 30 ml d'éther chlorhydrique (2M) et on laisse sous agitation pendant une nuit. Après filtration et séchage on obtient 9, 57 g de composé attendu.

Préparation 8.2 :

Chlorhydrate de 4-phénylpipéridine-4-carboxamide.

(V) : $R_1$ = 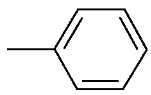

A) Chlorhydrate de 1-benzyl-4-phénylpipéridine-4-carboxamide.

Dans 77 ml d'acide sulfurique concentré, on ajoute 10 g de 1-benzyl-4-phényl-pipéridine-4-carbonitrile puis chauffe à 100°C pendant une heure. Le mélange réactionnel est ensuite ajouté sur de la glace puis basifié avec $NH_4OH$. Le produit est extrait au $CH_2Cl_2$, séché, évaporé. Après cristallisation du chlorhydrate dans l'éther, on obtient 5,7 g de produit attendu.

B) Chlorhydrate de 4-phénylpipéridine-4-carboxamide.

Dans 70 ml de MeOH, on ajoute 5.6 g de produit obtenu en (A), 6.7 g de cyclohexadiene, 0.5 g de palladium à 10% sur charbon et on chauffe le mélange réactionnel à reflux pendant 6 heures. On filtre le catalyseur sur de la célite et évapore à sec. Après cristallisation dans un mélange éther-éther isopropylique, on obtient 3 g de produit attendu.

## EXEMPLES

**Exemple 1: Composé N° 6**

1-{[4-(4-Chlorophényl)-5-(2,4-dichlorophényl)-3-(2-hydroxyéthoxy)thién-2-yl]carbonyl}-4-phénylpipéridine-4-carboxamide.

A) 1-({4-(4-Chlorophényl)-5-(2,4-dichlorophényl)-3-[2-(tétrahydro-2*H*-pyran-2-yloxy)éthoxy]thién-2-yl}carbonyl)-4-phénylpipéridine-4-carboxamide.

A un mélange de 0,264 g du composé de la Préparation 4.1 dans 20 ml d'acétone, on ajoute 0,188 g de 2-(2-bromoéthoxy)tétrahydro-2H-pyrane et 0,294 g de $Cs_2CO_3$. On chauffe à reflux pendant 18 heures. On filtre et concentre le filtrat sous vide. On purifie le brut réactionnel par chromatographie sur gel de silice en éluant au DCM puis par le mélange DCM/MeOH (97/3 ; v/v). On obtient 0,272 g du composé attendu.

B) 1-{[4-(4-Chlorophényl)-5-(2,4-dichlorophényl)-3-(2-hydroxyéthoxy)thién-2-yl]carbonyl}-4-phénylpipéridine-4-carboxamide.

A un mélange de 0,272 g du composé de l'étape précédente dans 15 ml d'acétone, on ajoute de la résine d'Amberlyst 15 puis chauffe à reflux pendant 2 heures. On filtre et concentre. On purifie le brut réactionnel par chromatographie sur gel de silice en éluant au DCM puis par le mélange DCM/MeOH (97/3 ; v/v). On obtient 0,146 g du composé attendu.

**Exemple 2 : Composé N° 4**

1-{[4-(4-chlorophényl)-5-(2,4-dichlorophényl)-3-(3-hydroxypropoxy)thién-2-yl]carbonyl}-4-phénylpipéridine-4-carboxamide

Ce composé est préparé en suivant un mode opératoire similaire à celui décrit aux étapes A) et B) décrits dans l'exemple 1, à partir du composé de la Préparation 4.1 et de 2-(3-bromopropoxy)tétrahydro-2*H*-pyrane.

**Exemple 3: Composé N° 5**

1-({4-(4-Chlorophényl)-5-(2,4-dichlorophényl)-3-[3-(méthylsulfonyl)propoxy] thién-2-yl}carbonyl)-4-phénylpipéridine-4-carboxamide.

A un mélange de 0,200 g du composé de la Préparation 4.1 dans 15 ml d'acétone, on ajoute 0,148 g de 3-(méthylsulfonyl)propylméthanesulfonate, 0,294 g de $CS_2CO_3$. On chauffe à reflux pendant 18 heures. On essore et concentre. On purifie le brut réactionnel par chromatographie sur gel de silice en éluant au DCM puis par le mélange DCM/MeOH (97/3 ; v/v). On obtient 0 ,065 mg du composé attendu.

**Exemple 4 : Composé N° 2**

1'-{[4-(4-Chlorophényl)-5-(2,4-dichlorophényl)-3-{3-[(méthylsulfonyl)amino]propoxy}thién-2-yl]carbonyl}-4-cyclohexylpipéridine-4-carboxamide

A) [3-({2-[(4-carbamoyl-4-cyclohexylpipéridin-1-yl)carbonyl]-4-(4-chlorophényl)-5-(2,4-dichlorophényl)thién-3-yl}oxy)propyl]carbamate de tert-butyle.

A un mélange de 0,300 g du composé de la Préparation 4.2 dans 20 ml d'acétone, on ajoute 0,212 g de (3-bromopropyl)carbamate de tert-butyle, 0,290 g de $CS_2CO_3$. On chauffe à reflux pendant 18 heures. On essore et concentre. On obtient 0,291 g du composé attendu.

B) 1-{[3-(3-aminopropoxy)-4-(4-chlorophényl)-5-(2,4-dichlorophényl)thién-2-yl]carbonyl}-4-cyclohexylpipéridine-4-carboxamide.

A un mélange de 0,291 g du composé de l'étape précédente dans 15 ml de DCM, on ajoute, goutte à goutte, 5 ml d'une solution 2 M d'éther chlorhydrique. On laisse sous agitation pendant une nuit à TA. On concentre à sec,

reprend dans l'éther éthylique, essore et sèche. On obtient 0,257 g du composé attendu.

C) 1-{[4-(4-chlorophényl)-5-(2,4-dichlorophényl)-3-{3-[(méthylsulfonyl)amino]propoxy}thién-2-yl]carbonyl}-4-cyclohexylpipéridine-4-carboxamide.

A un mélange de 0,257 mg du composé de l'étape précédente dans 10 ml de DCM, on ajoute, goutte à goutte, 0,04 ml d'une solution de chlorure de méthanesulfonyle, 0,18 ml d'une solution de triéthylamine et laisse sous agitation à TA pendant 1 heure. On évapore à sec, lave avec 15 ml d'eau distillée, extrait au DCM, sèche et concentre. On obtient 0,198 g de composé attendu.

**Exemple 5 : Composé N° 24**

Chlorhydrate de 4-(Benzylamino)-1-[[4-(4-chlorophényl)-5-(2,4-dichlorophényl)-3-(3-hydroxypropoxy)-2-thiényl]carbonyl]pipéridine-4-carboxamide.

A) 4-(Benzylamino)-1-[[4-(4-chlorophényl)-5-(2,4-dichlorophényl)-3-[3-(tétrahydro-2*H*-pyran-2-yloxy)propoxy]-2-thiényl]carbonyl]pipéridine-4-carboxamide.

On chauffe à reflux pendant une nuit un mélange de 0,35 g du composé de la Préparation 4.3, 0,153 g de 2-(3-bromopropoxy)tétrahydro-2*H*-pyrane et 0,22 g de $CS_2CO_3$ dans 15 ml d'acétone. On filtre le mélange réactionnel et concentre sous vide le filtrat. On chromatographie le résidu sur gel de silice en éluant au DCM puis par le gradient du mélange DCM/MeOH jusqu'à (75/25 ; v/v). On obtient 0 ,3 g du composé attendu.

B) Chlorhydrate de 4-(Benzylamino)-1-[[4-(4-chlorophényl)-5-(2,4-dichloro-phényl)-3-(3-hydroxypropoxy)-2-thiényl]carbonyl]pipéridine-4-carboxamide.

A un mélange de 0,3 g du composé de l'étape précédente dans 15 ml de MeOH, on ajoute 30 ml d'une solution d'éther chlorhydrique 2N et laisse 1 heure sous agitation à TA. On concentre le mélange réactionnel sous vide et chromatographie le résidu sur gel de silice en éluant par le mélange MeOH/$H_2O$ de (65/35 ; v/v) à (90/10 ; v/v). On obtient 0,18 g du composé attendu après cristallisation dans l'éther iso.

**Exemple 6 : Composé N° 26**

Chlorhydrate de 1-[5-(2-Chlorophényl)-4-(4-chlorophényl)-3-[3-(hydroxypropoxy)-2-thiényl]carbonyl]-4-[(4-fluorobenzyl)amino]pipéridine-4-carboxamide.

A) 1-[[5-(2-Chlorophényl)-4-(4-chlorophényl)-3-[3-(tétrahydro-2*H*-pyran-2-yloxy)propoxy]-2-thiényl]carbonyl]-4-[(4-fluorobenzyl)amino]pipéridine-4-carboxamide.

On laisse 1 heure sous agitation à TA un mélange de 0,3 g du composé de la Préparation 7.1, 0,19 g de dichlorohydrate de 4-[(4-fluorobenzyl)amino]pipéridine-4-carboxamide, 0,29 ml de triéthylamine et 0,19 g de TBTU dans 20 ml de DCM. On concentre le mélange réactionnel sous vide, reprend le résidu dans 70 ml de tampon PH = 2, extrait au DCM, sèche la phase organique sur $MgSO_4$, filtre et évapore le solvant sous vide. On chromatographie le résidu sur gel de silice en éluant au DCM puis par le gradient du mélange DCM/MeOH jusqu'à (70/30 ; v/v). On obtient 0,35 g du composé attendu.

B) Chlorhydrate de 1-[5-(2-Chlorophényl)-4-(4-chlorophényl)-3-[3-(hydroxypropoxy)-2-thiényl]carbonyl]-4-[(4-fluorobenzyl)amino]pipéridine-4-carboxamide.

A un mélange de 0,35 g du composé de l'étape précédente dans 30 ml de MeOH, on ajoute 30 ml d'une solution d'éther chlorhydrique 2N et laisse une nuit sous agitation à TA. On concentre le mélange réactionnel sous vide et chromatographie le résidu sur gel de silice en éluant par le mélange DCM/MeOH jusqu'à (60/40 ; v/v). On obtient 0,186 g du composé attendu après cristallisation dans l'éther iso.

**Exemple 7 : Composé N° 27**

1-[5-(2-Chlorophényl)-4-(4-chlorophényl)-3-[3-(hydroxypropoxy)-2-thiényl]carbonyl]-4-phénylpipéridine-4-carboxamide.

A) 1-[5-(2-Chlorophényl)-4-(4-chlorophényl)-3-[3-(tétrahydro-2*H*-pyran-2-yloxy)thiényl]carbonyl]-4-phénylpipéridine-4-carboxamide.

On laisse 1 heure sous agitation à TA un mélange de 0,26 g du composé de la Préparation 7.1, 0,13 g de chlorhydrate de 4-phénylpipéridine-4-carboxamide, 0,21 ml de triéthylamine et 0,165 g de TBTU dans 20 ml de DCM. On concentre le mélange réactionnel sous vide, reprend le résidu dans 70 ml de tampon PH = 2, extrait au DCM, sèche la phase organique sur $MgSO_4$, filtre et évapore le solvant sous vide. On chromatographie le résidu sur gel de silice en éluant au DCM puis par le gradient du mélange DCM/MeOH jusqu'à (60/40 ; v/v). On obtient 0,2 g du composé attendu.

B) 1-{[5-(2-Chlorophényl)-4-(4-chlorophényl)-3-(3-hydroxypropoxy)-2-thiényl]carbonyl}-4-phénylpipéridine-4-carboxamide

On chauffe à reflux pendant une nuit un mélange de 0,2 g du composé de l'étape précédente et de la résine d'Amberlyst 15 dans 30 ml de MeOH. On filtre le mélange réactionnel et concentre sous vide le filtrat. On chromatographie le résidu sur gel de silice en éluant au DCM puis par le gradient du mélange DCM/MeOH jusqu'à (60/40 ; v/v). On obtient 0,1 g du composé attendu.

Les tableaux 1 et 2 indiquent les structures chimiques de quelques composés selon l'invention ainsi que leurs propriétés physiques (analyse par couplage LC/UV/MS : chromatographie liquide/détection UV/spectrométrie de masse). Ces composés sont exemplifiés ci-avant ou préparés selon des modes opératoires similaires à ceux des composés exemplifiés (exemples 1 à 35).

Dans ces tableaux, Me représente un groupe méthyle.

TABLEAU 1

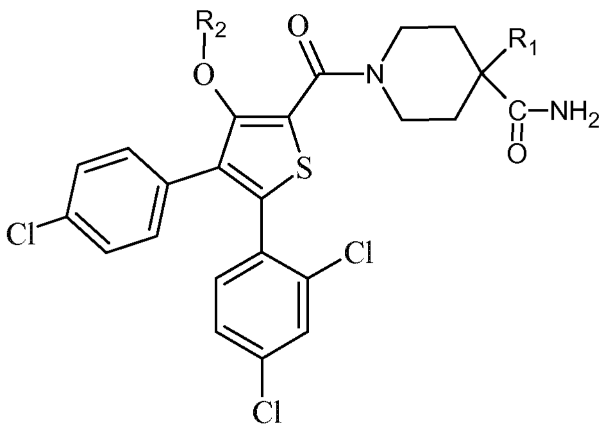

(I)

| Composés No | $R_1$ | $R_2$ | Caractérisation LC/MS $MH^+$ ; tr (min) (condition) |
|---|---|---|---|
| 1 | 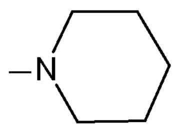 | -Me | $MH^+$ = 605<br>tr = 7,99<br>(A) |
| 2 | 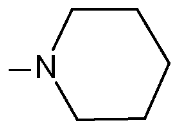 | $-(CH_2)_3$-NH-$SO_2$-Me | $MH^+$ = 726<br>tr = 7,8<br>(A) |

(suite)

| Composés No | $R_1$ | $R_2$ | Caractérisation LC/MS $MH^+$ ; tr (min) (condition) |
|---|---|---|---|
| 3 | 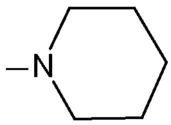 | $-(CH_2)_3-SO_2Me$ | $MH^+$ = 711<br>tr = 7,81<br>(A) |
| 4 | 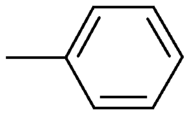 | $-(CH_2)_3-OH$ | $MH^+$ = 642<br>tr = 10,5<br>(A) |
| 5 | 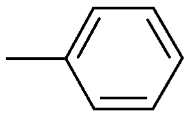 | $-(CH_2)_3-SO_2-Me$ | $MH^+$ = 704<br>tr = 10,5<br>(A) |
| 6 | 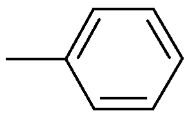 | $-(CH_2)_2-OH$ | $MH^+$ = 628<br>tr = 10,43<br>(A) |
| 7 | 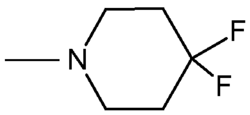 | $-(CH_2)_2-OH$ | $MH^+$ = 671<br>tr = 1,74<br>(C) |
| 8 | 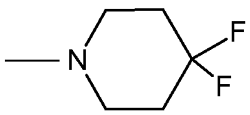 | $-(CH_2)_3-SO_2Me$ | $MH^+$ = 747<br>tr = 1,79<br>(C) |
| 9 | 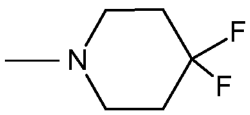 | $-(CH_2)_3-OH$ | $MH^+$ = 685<br>tr = 1,78<br>(C) |
| 10 | $-HN-(CH_2)_2-CF_3$ | $-(CH_2)_2-OH$ | $MH^+$ = 663<br>tr = 9,86<br>(B) |
| 11 | $-HN-(CH_2)_2-CF_3$ | $-(CH_2)_3-SO_2Me$ | $MH^+$ = 739<br>tr = 1,6<br>(C) |
| 12 | $-HN-(CH_2)_2-CF_3$ | $-(CH_2)_2-NH-CO-CH_2OH$ | $MH^+$ = 720<br>tr = 1,48<br>(C) |
| 13 | $-HN-(CH_2)_2-CF_3$ | $-(CH_2)_3-OH$ | $MH^+$ = 677<br>tr = 10,14<br>(B) |
| 14 | 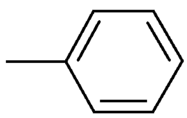 | $-(CH_2)_2-NH-CO-CH_2OH$ | $MH^+$ = 685<br>tr = 9,95<br>(A) |
| 15 | 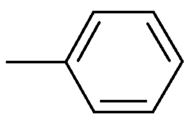 | $-(CH_2)_2-NH-CO-Me$ | $MH^+$ = 659<br>tr = 1,81<br>(C) |

(suite)

| Composés No | $R_1$ | $R_2$ | Caractérisation LC/MS MH⁺ ; tr (min) (condition) |
|---|---|---|---|
| 16 | 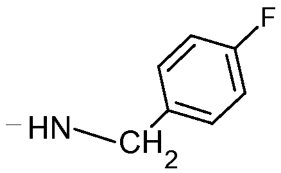 | $-(CH_2)_2$-OH | $MH^+$ = 675<br>tr = 1,56<br>(C) |
| 17 | 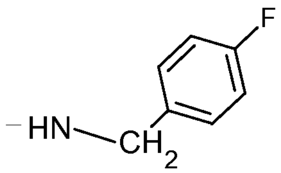 | $-(CH_2)_3$-OH | $MH^+$ = 689<br>tr = 1,58<br>(C) |
| 18 | 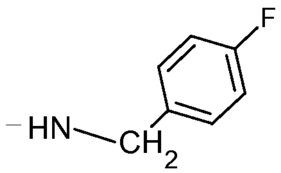 | $-(CH_2)_3-SO_2$-Me | $MH^+$ = 751<br>tr = 1,57<br>(C) |
| 19 | 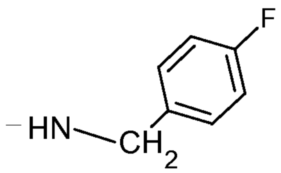 | $-(CH_2)_2$-NH-CO-Me | $MH^+$ = 716<br>tr = 1,53<br>(C) |
| 20 | 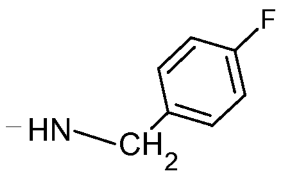 | $-(CH_2)_2$-NH-CO-$CH_2$OH | $MH^+$ = 732<br>tr = 1,48<br>(C) |
| 21 | 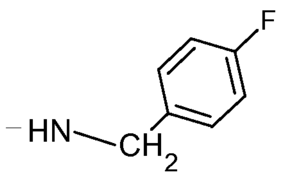 | $-(CH_2)_3$-NH-CO-$CH_2$OH | $MH^+$ = 746<br>tr = 1,53<br>(C) |
| 22 | 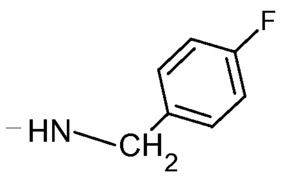 | $-(CH_2)_3$-NH-CO-Me | $MH^+$ = 730<br>tr = 1,59<br>(C) |
| 23 | 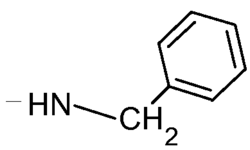 | $-(CH_2)_2$-OH | $MH^+$ = 557<br>tr = 1,55<br>(C) |
| 24 | 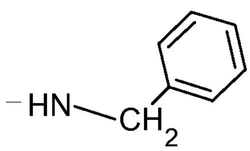 | $-(CH_2)_3$-OH | $MH^+$ = 671<br>tr = 1,56<br>(C) |

(suite)

| Composés No | $R_1$ | $R_2$ | Caractérisation LC/MS $MH^+$ ; tr (min) (condition) |
|---|---|---|---|
| 25 | 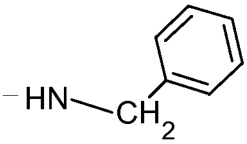 | $-(CH_2)_2$-NH-CO-$CH_2OH$ | $MH^+$ = 714<br>tr = 1,46<br>(C) |

TABLEAU 2

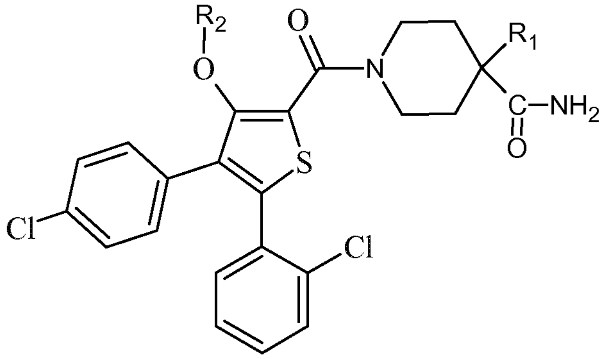

(I)

| Composés No | $R_1$ | $R_2$ | Sel | Caractérisation LC/MS $MH^+$ ; tr (min) (condition) |
|---|---|---|---|---|
| 26 | 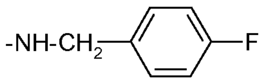 | $-(CH_2)_3$-OH | HCl | $MH^+$ = 655<br>tr = 1,43<br>(C) |
| 27 | 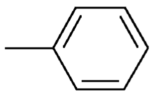 | $-(CH_2)_3$-OH | - | $MH^+$ = 608<br>tr = 1,72<br>(C) |
| 28 | 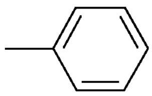 | $-(CH_2)_2$-OH | - | $MH^+$ = 594<br>tr = 1,7<br>(C) |
| 29 | 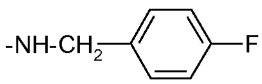 | $-(CH_2)_2$-OH | HCl | $MH^+$ = 641<br>tr = 1,41<br>(C) |
| 30 | 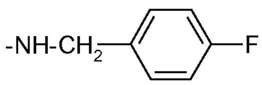 | $-(CH_2)_2-NH_2$ | HCl | $MH^+$ = 640<br>tr= 1,18<br>(C) |
| 31 | 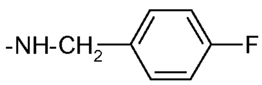 | $-(CH_2)_2-NHCOCH_3$ | - | $MH^+$ = 682<br>tr = 1,38<br>(C) |
| 32 | 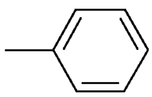 | $-(CH_2)_2-NH_2$ | HCl | $MH^+$ = 593<br>tr = 8,42<br>(B) |
| 33 | 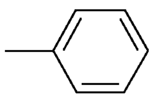 | $-(CH_2)_2-NHCOCH_2OH$ | - | $MH^+$ = 651<br>tr = 8,79<br>(B) |

(suite)

| Composés No | $R_1$ | $R_2$ | Sel | Caractérisation LC/MS $MH^+$ ; tr (min) (condition) |
|---|---|---|---|---|
| 34 | 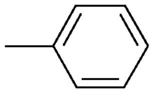 | $-(CH_2)_2-NHCOCH_3$ | - | $MH^+$ = 635<br>tr = 1,64<br>(C) |
| 35 | $-NH-CH_2$ 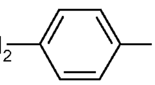 F | $-(CH_2)_2-NHCOCH_2OH$ | - | $MH^+$ = 699<br>tr = 1,33<br>(C) |

Les analyses effectuées par RMN pour les composés 24, 26 et 27 sont données ci-après :

Composé 24 :RMN $^1H$: DMSO-$d_6$ (250 MHz) : δ (ppm) : 1,53 - 1,68 : m : 2H ; 1,68 - 2,01 : m : 4H ; 3,25 - 3,34 : m : 4H ; 3,42 - 3,98 : m : 4H ; 3,87 : t : 2H ; 4,34 : t : 1H ; 7,10 - 7,21 : m : 3H ; 7,22 - 7,51 : m : 11H ; 7,68 : d : 1H.
Composé 26 : RMN $^1H$: DMSO-$d_6$ (250 MHz) : δ (ppm) : 1,54 - 1,68 : m : 2H ; 1,68 - 2,19 : m : 2H ; 2,33 - 2,58 : m : 2H ; 3,20 - 3,40 : m : 4H ; 3,44 - 3,71 : m : 2H ; 3,87 : t : 2H ; 3,95 - 4,22 : m : 2H ; 4,39 : br.s. : 1H ; 7,17 : d : 3H ; 7,22 - 7,54 : m : 9H ; 7,65 : br.s. : 1H ; 8,06 : br.s. : 1H ; 9,62 : br.s. : 1H.
Composé 27 : RMN $^1H$: DMSO-$d_6$ (250 MHz) : δ (ppm) : 1,46 - 1,65 : m : 2H ; 1,74 - 1,96 : m : 2H ; 2,50 - 2,63 : m : 2H ; 3,25 : br.s. : 2H ; 3,21 - 3,30 : m : 2H ; 3,85 : t : 2H ; 4,7 : br.s. : 2H ; 4,35 : t : 1H ; 7,11 : s : 1H ; 7,17 : d : 2H ; 7,22 - 7,32 : m : 2H ; 7,32 - 7,57 : m : 10H.

Les composés de formule (I) possèdent une très bonne affinité *in vitro* ($IC_{50} \leq 5.10^{-7}$ M) pour les récepteurs aux cannabinoïdes $CB_1$, dans les conditions expérimentales décrites par M. Rinaldi-Carmona et al. (FEBS Letters, 1994, 350, 240-244).

La nature antagoniste des composés de formule (I) a été démontrée *in vitro* par les résultats obtenus dans les modèles d'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

La faible pénétration des composés de formule (I) au niveau de la barrière hématoencéphalique (BHE) a été évaluée *in vivo* par :

- Mesure (1) : quantification des composés de formule (I) (inchangé) dans des échantillons de cerveau de souris après une administration par voie intraveineuse ou orale, à l'aide de technique analytique (LC-MS/MS).

$$\text{Le ratio } \frac{\text{quantité présente dans le cerveau}}{\text{quantité présente dans le plasma}} \text{ inférieur à 0,2 traduit une faible}$$

pénétration du composé au niveau du cerveau.

- Mesure (2) : mesure de l'interaction des composés de formule (I) avec les récepteurs CB1 présents dans le cerveau chez la souris à l'aide d'un test de binding *ex vivo* du [3H]-CP55940 (agoniste CB1) après une administration par voie intraveineuse (10 mg/kg) comme décrit dans M. Rinaldi-Carmona et al., FEBS Letters, 1994, 350, 240-244 et M. Rinaldi-Carmona et al., Life Sciences, 1995, 56, 1941-1947, M. Rinaldi-Carmona et al., J. Pharmacol. Exp. Ther., 2004, 310, 905-914.
  Un pourcentage d'inhibition de la liaison du [3H]-CP55940 au niveau du cerveau inférieur à 50% à 10 mg/kg traduit une faible pénétration au niveau du cerveau. De préférence, ce pourcentage est inférieur à 40% et plus préférentiellement inférieur à 30%.
- Mesure (3) : la mesure du blocage par les composés de formule (I) de l'effet hypothermique induit par un agoniste des récepteurs CB1 (CP55940), après une administration par voie intraveineuse, comme décrit dans Rinaldi-Carmona M. et al., JPET 2004, 310, 905-914).
  Un pourcentage de réversion de l'effet du CP55940 inférieure à 50 % à 10 mg/kg traduit une faible pénétration au niveau du cerveau. De préférence, ce pourcentage est inférieur à 40% et plus préférentiellement inférieur à 30%.

L'interaction des composés de formule (I) selon l'invention avec les récepteurs CB1 présents à la périphérie a été démontrée chez la souris par la mesure du blocage de l'effet inhibiteur induit par un agoniste des récepteurs CB1 (CP55940) sur le transit gastro-intestinal, après une administration orale, comme décrit dans M. Rinaldi-Carmona et al., J. Pharmacol. Exp. Ther., 2004, 310, 905-914). Un pourcentage de réversion de l'effet du CP55940 supérieur à 50 % à 10 mg/kg traduit un pouvoir antagoniste significatif au niveau des récepteurs CB1 présent à la périphérie. De préférence, le pourcentage de réversion est compris entre 70% et 100%.

Les composés de formule (I) sont compatibles avec leur utilisation en tant que médicament.

Ainsi, selon un autre de ses aspects, l'invention a pour objet des médicaments pour la médecine humaine ou vétérinaire qui comprennent un composé de formule (I), ou un sel d'addition de ce dernier à un acide pharmaceutiquement acceptable.

Ainsi les composés selon l'invention peuvent être utilisés chez l'homme ou chez l'animal (notamment chez les mammifères incluant de façon non limitative les chiens, les chats, les chevaux, les bovins, les moutons) dans le traitement ou la prévention de maladies impliquant les récepteurs aux cannabinoïdes $CB_1$.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles du déficit de l'attention et de l'hyperactivité (TDAH) chez les enfants hyperkinétiques ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance.

De plus, les composés de formule (I) peuvent être utiles comme neuroprotecteurs, dans le traitement de l'ischémie, des traumatismes crâniens et le traitement des maladies neurodégénératives aiguës ou chroniques : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans la prévention et le traitement des troubles de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement de l'obésité et des risques associés à l'obésité, notamment les risques cardiovasculaires.

De plus, les composés de formule (I) selon l'invention peuvent être utilisés en tant que médicaments dans le traitement et la prévention des troubles gastro-intestinaux, des troubles diarrhéiques, des ulcères, des vomissements, des troubles vésicaux et urinaires, des maladies du foie d'origine alcoolique ou non telles que la cirrhose chronique, la fibrose, la stéatose hépatique, la stéatohépatite ; ainsi que des troubles d'origine endocrinienne, des troubles cardiovasculaires, de l'hypotension, de l'athérosclérose, du choc hémorragique, du choc septique, de l'asthme, de la bronchite chronique, des maladies pulmonaires chroniques obstructives, du syndrome de Raynaud, du glaucome, des troubles de la fertilité, de l'accouchement prématuré, de l'interruption de grossesse, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement des maladies des os et de l'ostéoporose. De plus, les composés de formule (I) selon l'invention peuvent être utilisés pour leurs effets protecteurs contre la cardiotoxicité induite par les médicaments.

Selon la présente invention, les composés de formule (I) sont tout particulièrement utiles pour la préparation de médicaments utiles pour la prévention et le traitement des désordres psychiatriques, en particulier la schizophrénie, les troubles de l'attention et de la vigilance, les troubles du déficit de l'attention et de l'hyperactivité (TDAH) chez les enfants hyperkinétiques; pour la prévention et le traitement des déficits mnésiques et des troubles cognitifs ; de la dépendance et du sevrage à une substance, en particulier la dépendance alcoolique, la dépendance nicotinique, le sevrage alcoolique et le sevrage tabagique ; des maladies neurodégénératives aiguës ou chroniques.

Plus particulièrement, les composés de formule (I) selon la présente invention sont utiles dans la préparation

de médicaments utiles dans le traitement et la prévention des troubles de l'appétit, les troubles de l'appétence, des troubles du métabolisme, l'obésité, le diabète de type II, le syndrome métabolique, la dyslipidémie, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I), de ses sels pharmaceutiquement acceptables pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | : 50,0 mg |
| Mannitol | : 223,75 mg |
| Croscarmellose sodique | : 6,0 mg |
| Amidon de maïs | : 15,0 mg |
| Hydroxypropyl-méthylcellulose | : 2,25 mg |
| Stéarate de magnésium | : 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

**1.** Composé de formule (I) :

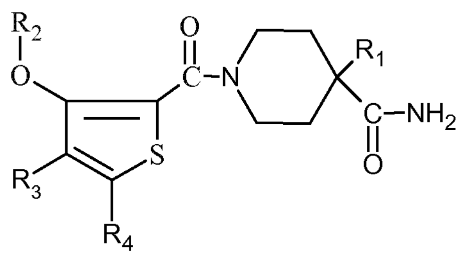

(I)

dans laquelle :

- $R_1$ représente :

. un groupe $-NR_5R_6$ ;
. un phényle non substitué ou substitué une ou plusieurs fois par des substituants choisis indépendamment parmi un atome d'halogène, un $(C_1-C_4)$alkyle, un $(C_1-C_4)$alcoxy, un trifluorométhyle ;

- $R_2$ représente :

. un $(C_1-C_4)$alkyle ;
. un groupe $-X-R_7$ ;

- $R_3$ et $R_4$ représentent chacun indépendamment un phényle substitué une ou plusieurs fois par un substituant choisi indépendamment parmi un atome d'halogène, un $(C_1-C_4)$alkyle, un $(C_1-C_4)$alcoxy, un trifluorométhyle ; :
- $R_5$ représente un atome d'hydrogène ou un $(C_1-C_4)$alkyle ;
- $R_6$ représente un $(C_1-C_4)$alkyle non substitué ou substitué par un ou plusieurs atomes de fluor ou par un phényle non susbtitué ou substitué par un atome d'halogène ;
- ou bien $R_5$ et $R_6$ ensemble avec l'atome d'azote auquel ils sont liés constituent un hétérocycle choisi parmi : l'azétidine, la pyrrolidine, la pipéridine, ledit hétérocycle étant non substitué ou subtitué une ou plusieurs fois par un atome d'halogène ;
- X représente un $(C_1-C_5)$alkylène ;
- $R_7$ représente un groupe $-OR_8$, un groupe $-NR_9R_{10}$, un groupe $-SO_2-(C_1-C_4)$alkyle ;
- $R_8$ représente un atome d'hydrogène ou un $(C_1-C_4)$alkyle ;
- $R_9$ représente un atome d'hydrogène ou un $(C_1-C_4)$alkyle ;
- $R_{10}$ représente un atome d'hydrogène, un groupe $-COR_{11}$, un groupe $-SO_2R_{11}$ ou un groupe $-CO(CH_2)_mOH$ ;
- $R_{11}$ représente un $(C_1-C_4)$alkyle non substitué ou substitué par un ou plusieurs atomes de fluor ;
- m représente 1, 2 ou 3 ;

à l'état de base ou de sel d'addition à un acide.

**2.** Composé de formule (I), selon la revendication 1, dans laquelle :

- $R_1$ représente :

. un groupe $-NR_5R_6$ ;
. un phényle ;

- $R_2$ représente :

. un méthyle ;
. un groupe $-X-R_7$ ;

- $R_3$ et $R_4$ représentent chacun indépendamment un phényle substitué une ou deux fois par un atome d'halogène ;
- $R_5$ représente un atome d'hydrogène ;
- $R_6$ représente un $(C_1-C_4)$alkyle substitué par un ou plusieurs atomes de fluor ou par un phényle non substitué ou substitué par un atome d'halogène ;
- ou bien $R_5$ et $R_6$ ensemble avec l'atome d'azote auquel ils sont liés constituent un radical pipéridin-1-yle non substitué ou substitué une ou deux fois par un atome d'halogène ;
- X représente un $(C_1-C_3)$alkylène ;
- $R_7$ représente un groupe $-OR_8$, un groupe $-NR_9R_{10}$, un groupe $-SO_2-CH_3$ ;
- $R_8$ représente un atome d'hydrogène ;
- $R_9$ représente un atome d'hydrogène ;
- $R_{10}$ représente un atome d'hydrogène, un groupe $-CO-CH_3$, un groupe $-SO_2-CH_3$ ou un groupe $-COCH_2OH$ ;

à l'état de base ou de sel d'addition à un acide.

**3.** Composé de formule (I) selon la revendication 1 dans laquelle :

- $R_1$ représente :

. un groupe $-NH(CH_2)_2-CF_3$, un groupe 4-fluorobenzylamino, un groupe benzylamino, un radical pipéridin-1-yle, un radical 4,4-difluoropipéridin-1-yle ;
. un phényle ;

- $R_2$ représente :

. un méthyle ;
. un groupe $-X-R_7$ ;

- $R_3$ représente un 4-chlorophényle ;
- $R_4$ représente un 2-chlorophényle, un 2,4-dichlorophényle ;
- X représente un méthylène, un éthylène, un triméthylène ;
- $R_7$ représente un radical -OH, un radical $NH_2$ ,un groupe $-NHCOCH_3$, un groupe $-NHSO_2CH_3$, un groupe $-NHCOCH_2OH$, un groupe $-SO_2-CH_3$ ;

à l'état de base ou de sel d'addition à un acide.

**4.** Composé selon la revendication 1 choisi parmi :

- 1'-{[4-(4-chlorophényl)-5-(2,4-dichlorophényl)-3-méthoxythién-2-yl]carbonyl}-1,4'-bipipéridine-4'-carboxamide; - 1'-{[4-(4-chlorophényl)-5-(2,4-dichlorophényl)-3-{3-[(méthylsulfonyl) amino]propoxy}thién-2-yl]carbonyl}-1,4'-bipéridine-4'-carboxamide ;
- 1-({4-(4-chlorophényl)-5-(2,4-dichlorophényl)-3-[3-(méthylsulfonyl)propoxy] thién-2-yl}carbonyl)-1,4'-bipipéridine-4'-carboxamide ;
- 1-{[4-(4-chlorophényl)-5-(2,4-dichlorophényl)-3-(3-hydroxypropoxy)thién-2-yl]carbonyl}-4-phénylpipéridine-4-carboxamide ;
- 1-({4-(4-chlorophényl)-5-(2,4-dichlorophényl)-3-[3-(méthylsulfonyl)propoxy] thién-2-yl}carbonyl)-4-phénylpipéridine-4-carboxamide ;
- 1-{[4-(4-chlorophényl)-5-(2,4-dichlorophényl)-3-(2-hydroxyéthoxy)thién-2-yl]carbonyl}-4-phénylpipéridine-4-carboxamide ;
- 1'-{[4-(4-chlorophényl)-5-(2,4-dichlorophényl)-3-(2-hydroxyéthoxy)thién-2-yl]carbonyl}-4,4-difluoro-1,4'-bipipéridine-4'-carboxamide ;
- 1'-{[4-(4-chlorophényl)-5-(2,4-dichlorophényl)-3-(3-hydroxypropoxy)thién-2-yl]carbonyl}-4,4-difluoro-1,4'-bipipéridine-4'-carboxamide ;
- 1-{[4-(4-chlorophényl)-5-(2,4-dichlorophényl)-3-(2-hydroxyéthoxy)thién-2-yl]carbonyl}-4-[(3,3,3-trifluoropropyl)amino]pipéridine-4-carboxamide ;
- 1-({4-(4-chlorophényl)-5-(2,4-dichlorophényl)-3-[3-(méthylsulfonyl) propoxy]thién-2-yl}carbonyl)-4-[(3,3,3-trifluoropropyl)amino]pipéridine-4-carboxamide ;
- 1-{[4-(4-chlorophényl)-5-(2,4-dichlorophényl)-3-{2(hydroxyacétyl)amino]éthoxy}thién-2-yl]carbonyl}-4-[(3,3,3-trifluoropropyl)amino]pipéridine-4-carboxamide ;
- 1-{[4-(4-chlorophényl)-5-(2,4-dichlorophényl)-3-(3-hydroxypropoxy)thién-2-yl]carbonyl}-4-[(3,3,3-trifluoropropyl)amino]pipéridine-4-carboxamide ;
- 1-{[4-(4-chlorophényl)-5-(2,4-dichlorophényl)-3-{2-[(hydroxyacétyl)amino]éthoxy}thién-2-yl]carbonyl}-3-phénylpipéridine-4-carboxamide ;
- 1-{[3-(2-acétamidoéthoxy)-4-(4-chlorophényl)-5-(2,4-dichlorophényl)thién-2-yl]carbonyl}-4-phénylpipéridine-4-carboxamide ;
- 1-{[4-(4-chlorophényl)-5-(2,4-dichlorophényl)-3-(2-hydroxyéthoxy)thién-2-yl]carbonyl}-4-[(4-fluorobenzyl)amino]pipéridine-4-carboxamide ;
- 1-{[4-(4-chlorophényl)-5-(2,4-dichlorophényl)-3-(3-hydroxypropoxy)thién-2-yl]carbonyl}-4-[(4-fluorobenzyl)amino]pipéridine-4-carboxamide ;
- 1-({4-(4-chlorophényl)-5-(2,4-dichlorophényl)-3-[3-(méthylsulfonyl)propoxy] thién-2-yl}carbonyl)-4-[(4-fluorobenzyl)amino]pipéridine-4-carboxamide ;
- 1-{[3-(2-acétamidoéthoxy)-4-(4-chlorophényl)-5-(2,4-dichlorophényl)thién-2-yl]carbonyl}-4-[(4-fluorobenzyl)amino]pipéridine-4-carboxamide ;
- 1-{[4-(4-chlorophényl)-5-(2,4-dichlorophényl)-3-{2-[(hydroxyacétyl)amino] éthoxy}thién-2-yl]carbonyl}-4-[(4-fluorobenzyl)amino]pipéridine-4-carboxamide ;
- 1-{[4-(4-chlorophényl)-5-(2,4-dichlorophényl)-3-{3-[(hydroxyacétyl)amino] propoxy}thién-2-yl]carbonyl}-4-[(4-fluorobenzyl)amino]pipéridine-4-carboxamide ;

- 1-{[3-(3-acétamidopropoxy)-4-(4-chlorophényl)-5-(2,4-dichlorophényl)thién-2-yl]carbonyl}-4-[(4-fluorobenzyl)amino]pipéridine-4-carboxamide ;
- 4-(benzylamino)-1-{[4-(4-chlorophényl)-5-(2,4-dichlorophényl)-3-(2-hydroxyéthoxy)thién-2-yl]carbonyl}pipéridine-4-carboxamide ;
- 4-(benzylamino)-1-{[4-(4-chlorophényl)-5-(2,4-dichlorophényl)-3-{2-[(hydroxyacétyl)amino]éthoxy}thién-2-yl]carbonyl}pipéridine-4-carboxamide ;
- 1-{[5-(2-chlorophényl)-4-(4-chlorophényl)-3-(3-hydroxypropoxy)thién-2-yl]carbonyl}-4-[(4-fluorobenzyl)amino]pipéridine-4-carboxamide ;
- 1-{[5-(2-chlorophényl)-4-(4-chlorophényl)-3-(3-hydroxypropoxy)thién-2-yl]carbonyl}-4-phénylpipéridine-4-carboxamide ;
- 1-{[5-(2-chlorophényl)-4-(4-chlorophényl)-3-(2-hydroxyéthoxy)thién-2-yl]carbonyl}-4-phénylpipéridine-4-carboxamide ;
- 1-{[5-(2-chlorophényl)-4-(4-chlorophényl)-3-(2-hydroxyéthoxy)thién-2-yl]carbonyl}-4-[(4-fluorobenzyl)amino]pipéridine-4-carboxamide ;
- 1-{[3-(2-aminoéthoxy)-5-(2-chlorophényl)-4-(4-chlorophényl)thién-2-yl]carbonyl}-4-[(4-fluorobenzyl)amino]pipéridine-4-carboxamide ;
- 1-{[3-(2-acétamidoéthoxy)-5-(2-chlorophényl)-4-(4-chlorophényl)thién-2-yl]carbonyl}-4-[(4-fluorobenzyl)amino]pipéridine-4-carboxamide ;
- 1-{[3-(2-aminoéthoxy)-5-(2-chlorophényl)-4-(4-chlorophényl)thién-2-yl]carbonyl}-4-phénylpipéridine-4-carboxamide ;
- 1-{[5-(2-chlorophényl)-4-(4-chlorophényl)-3-{2-[(hydroxyacétyl)amino]éthoxy}thién-2-yl]carbonyl}-4-phénylpipéridine-4-carboxamide ;
- 1-{[3-(2-acétamidoéthoxy)-5-(2-chlorophényl)-4-(4-chlorophényl)thién-2-yl]carbonyl}-4-phénylpipéridine-4-carboxamide ;
- 1-{[5-(2-chlorophényl)-4-(4-chlorophényl)-3-{2-(hydroxyacétyl)amino]éthoxy} thién-2-yl]carbonyl}-4-[(4-fluorobenzyl)amino]pipéridine-4-carboxamide ;

à l'état de base ou de sel d'addition à un acide.

**5.** Procédé pour la préparation d'un composé de formule (I) selon la revendication 1, **caractérisé en ce que** :

on fait réagir, en présence d'une base, un composé de formule :

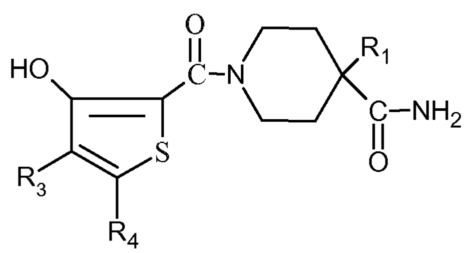

(II)

dans laquelle $R_1$, $R_3$ et $R_4$ sont tels que définis pour un composé de formule (I) selon la revendication 1, avec un composé de formule :

$Z-R_2$ (III)

dans laquelle $R_2$ est tel que défini pour un composé de formule (I) selon la revendication 1 et Z représente un groupe partant.

**6.** Procédé pour la préparation d'un composé de formule (I) selon la revendication 1, **caractérisé en ce que** :

on fait réagir un acide ou un dérivé fonctionnel de cet acide de formule :

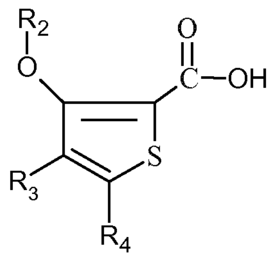

(IV)

dans laquelle $R_2$, $R_3$ et $R_4$ sont tels que définis pour un composé de formule (I) selon la revendication 1, avec un composé de formule :

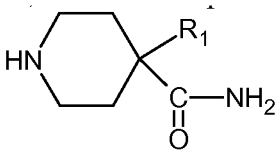

(V)

dans laquelle $R_1$ est tel que défini pour un composé de formule (I) selon la revendication 1.

7. Médicament, **caractérisé en ce qu'**il comprend un composé de formule (I) selon l'une quelconque des revendications 1 à 4, ou un sel d'addition de ce composé de formule (I) à un acide pharmaceutiquement acceptable.

8. Composition pharmaceutique, **caractérisée en ce qu'**elle comprend un composé de formule (I) selon l'une quelconque des revendications 1 à 4 ou un sel d'addition de ce composé de formule (I) à un acide pharmaceutiquement acceptable ainsi qu'au moins un excipient pharmaceutiquement acceptable.

9. Utilisation d'un composé selon l'une quelconque des revendications 1 à 4 pour la préparation d'un médicament destiné au traitement et/ou prévention des désordres psychiatriques, de la dépendance et du sevrage à une substance, des troubles cognitifs, des troubles de l'attention et de la vigilance, des maladies neurodégénératives aiguës et chroniques.

10. Utilisation d'un composé selon l'une quelconque des revendications 1 à 4 pour la préparation d'un médicament destiné au traitement et/ou prévention des troubles du métabolisme, des troubles de l'appétit, des troubles de l'appétence, de l'obésité, de la boulimie, du diabète, du syndrome métabolique, de la dyslipidémie.

11. Utilisation d'un composé selon l'une quelconque des revendications 1 à 4 pour la préparation d'un médicament destiné au traitement et/ou prévention de la douleur, des douleurs neuropathiques, des douleurs aiguës périphériques, des douleurs chroniques d'origine inflammatoire, des douleurs induites par un traitement anticancéreux.

12. Utilisation d'un composé selon l'une quelconque des revendications 1 à 4 pour la préparation d'un médicament destiné au traitement et/ou prévention des troubles gastro-intestinaux, des vomissements, des troubles diarrhéiques, des ulcères, des maladies du foie.

13. Utilisation d'un composé selon l'une quelconque des revendications 1 à 4 pour la préparation d'un médicament destiné au traitement et/ou prévention des phénomènes inflammatoires, des maladies du système immunitaire, de l'arthrite rhumatoïde, des maladies entrainant une démyélinisation, de la sclérose en plaque, des maladies des os et de l'ostéoporose.

14. Utilisation d'un composé selon l'une quelconque des revendications 1 à 4 pour la préparation d'un médicament destiné au traitement et/ou prévention des maladies et troubles du mouvement, en particulier des dyskinésies ou de la maladie de Parkinson, des démences séniles et de la maladie de Alzheimer.

## Patentansprüche

1. Verbindungen der Formel (I):

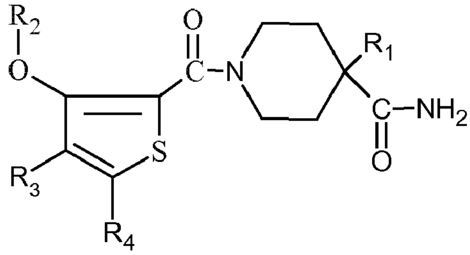

worin:

- $R_1$ für Folgendes steht:
  - eine Gruppe $-NR_5R_6$;
  - ein Phenyl, das nicht substituiert oder einfach oder mehrfach mit Substituenten, unabhängig ausgewählt aus einem Halogenatom, einem $(C_1-C_4)$Alkyl, einem $(C_1-C_4)$Alkoxy, einem Trifluormethyl, substituiert ist;
- $R_2$ für Folgendes steht:
  - ein $(C_1-C_4)$-Alkyl,
  - eine Gruppe $-X-R_7$;
- $R_3$ und $R_4$ jeweils unabhängig für ein Phenyl stehen, das einfach oder mehrfach mit einem Substituenten, unabhängig ausgewählt aus einem Halogenatom, einem $(C_1-C_4)$Alkyl, einem $(C_1-C_4)$Alkoxy, einem Trifluormethyl, substituiert ist;
- $R_5$ für ein Wasserstoffatom oder ein $(C_1-C_4)$Alkyl steht;
- $R_6$ für ein $(C_1-C_4)$Alkyl steht, das nicht substituiert oder mit einem oder mehreren Fluoratomen substituiert ist, oder mit einem Phenyl, das nicht substituiert oder mit einem Halogenatom substituiert ist;
- oder $R_5$ und $R_6$, zusammen mit dem Stickstoffatom, an das sie gebunden sind, einen Heterocyclus bilden, ausgewählt aus: Azetidin, Pyrrolidin, Piperidin, wobei der Heterocyclus nicht substituiert oder einfach oder mehrfach mit einem Halogenatom substituiert ist;
- X für ein $(C_1-C_5)$Alkylen steht;
- $R_7$ für eine Gruppe $-OR_8$, eine Gruppe $-NR_9R_{10}$, eine Gruppe $-SO_2-(C_1-C_4)$Alkyl steht;
- $R_8$ für ein Wasserstoffatom oder ein $(C_1-C_4)$Alkyl steht;
- $R_9$ für ein Wasserstoffatom oder ein $(C_1-C_4)$Alkyl steht;
- $R_{10}$ für ein Wasserstoffatom, eine Gruppe $-COR_{11}$, eine Gruppe $-SO_2R_{11}$ oder eine Gruppe $-CO(CH_2)_mOH$ steht;
- $R_{11}$ für ein $(C_1-C_4)$Alkyl steht, das nicht substituiert oder einfach oder mehrfach mit Fluoratomen substituiert ist;
- m für 1, 2 oder 3 steht;

in Form einer Base oder eines Säureadditionssalzes.

2. Verbindung der Formel (I) nach Anspruch 1, worin:

- $R_1$ für Folgendes steht:
  - eine Gruppe $-NR_5R_6$;
  - ein Phenyl;
- $R_2$ für Folgendes steht:
  - ein Methyl;
  - eine Gruppe $-X-R_7$;

- $R_3$ und $R_4$ jeweils unabhängig für ein Phenyl stehen, das einfach oder zweifach mit einem Halogenatom substituiert ist;
- $R_5$ für ein Wasserstoffatom steht,
- $R_6$ für ein $(C_1-C_4)$Alkyl steht, das mit einem oder mehreren Fluoratomen substituiert ist, oder mit einem Phenyl, das nicht substituiert oder mit einem Halogenatom substituiert ist;
- oder $R_5$ und $R_6$, zusammen mit dem Stickstoffatom, an das sie gebunden sind, einen Piperidin-1-yl-Rest bilden, der nicht substituiert oder einfach oder zweifach mit einem Halogenatom substituiert ist;
- X für ein $(C_1-C_3)$Alkylen steht;
- $R_7$ für eine Gruppe $-OR_8$, eine Gruppe $-NR_9R_{10}$, eine Gruppe $-SO_2-CH_3$ steht;
- $R_8$ für ein Wasserstoffatom steht,
- $R_9$ für ein Wasserstoffatom steht,
- $R_{10}$ für ein Wasserstoffatom, eine Gruppe $-CO-CH_3$, eine Gruppe $-SO_2-CH_3$ oder eine Gruppe $-COCH_2OH$ steht;

in Form einer Base oder eines Säureadditionssalzes.

**3.** Verbindung der Formel (I) nach Anspruch 1, worin:

- $R_1$ für Folgendes steht:

  · eine Gruppe $-NH\ (CH_2)\ _2-CF_3$, eine 4-Fluorbenzylaminogruppe, eine Benzylaminogruppe, einen Piperidin-1-yl-Rest, einen 4,4-Difluorpiperidin-1-yl-Rest;
  · ein Phenyl;

- $R_2$ für Folgendes steht:

  · ein Methyl;
  · eine Gruppe $-X-R_7$;

- $R_3$ für ein 4-Chlorphenyl steht;
- $R_4$ für ein 2-Chlorphenyl, ein 2,4-Dichlorphenyl steht;
- X für ein Methylen, ein Ethylen ein Trimethylen steht;
- $R_7$ für einen Rest -OH, einen Rest $NH_2$, eine Gruppe $-NHCOCH_3$, eine Gruppe $-NHSO_2CH_3$, eine Gruppe $-NHCOCH_2OH$, eine Gruppe $-SO_2-CH_3$ steht;

in Form einer Base oder eines Säureadditionssalzes.

**4.** Verbindung nach Anspruch 1, ausgewählt aus:

- 1'{[4-(4-Chlorphenyl)-5-(2,4-dichlorphenyl)-3-methoxythien-2-yl]carbonyl}-1,4'-bipiperidin-4'-carboxamid;
- 1'-{[4-(4-Chlorphenyl)-5-(2,4-dichlorphenyl)-3-{3-[(methylsulfonyl)amino]propoxy}thien-2-yl]carbonyl}-1,4'-bipiperidin-4'-carboxamid;
- 1'-({4-(4-Chlorphenyl)-5-(2,4-dichlorphenyl)-3-[3-(methylsulfonyl)propoxy]thien-2-yl}carbonyl)-1,4'-bipiperidin-4'-carboxamid;
- 1-{[4-(4-Chlorphenyl)-5-(2,4-dichlorphenyl)-3-(3-hydroxypropoxy)thien-2-yl]carbonyl}-4-phenylpiperidin-4-carboxamid;
- 1-({4-(4-Chlorphenyl)-5-(2,4-dichlorphenyl)-3-[3-(methylsulfonyl)propoxy]thien-2-yl}carbonyl)-4-phenylpiperidin-4-carboxamid;
- 1-{[4-(4-Chlorphenyl)-5-(2,4-dichlorphenyl)-3-(2-hydroxyethoxy)thien-2-yl]carbonyl}-4-phenylpiperidin-4-carboxamid;
- 1'-{[4-(4-Chlorphenyl)-5-(2,4-dichlorphenyl)-3-(2-hydroxyethoxy)thien-2-yl]carbonyl}-4,4-difluor-1,4'-bipiperidin-4'-carboxamid;
- 1'-{[4-(4-Chlorphenyl)-5-(2,4-dichlorphenyl)-3-(3-hydroxypropoxy)thien-2-yl]carbonyl}-4,4-difluor-1,4'-bipiperidin-4'-carboxamid;
- 1-{[4-(4-Chlorphenyl)-5-(2,4-dichlorphenyl)-3-(2-hydroxyethoxy)thien-2-yl]carbonyl}-4-[(3,3,3-trifluorpropyl)amino]piperidin-4-carboxamid;
- 1-({4-(4-Chlorphenyl)-5-(2,4-dichlorphenyl)-3-[3-(methylsulfonyl)propoxy]thien-2-yl}carbonyl)-4-[(3,3,3-trifluorpropyl)amino]piperidin-4-carboxamid;

- 1-{[4-(4-Chlorphenyl)-5-(2,4-dichlorphenyl)-3-{2(hydroxyacetyl)amino]ethoxy}thien-2-yl]carbonyl}-4-[(3,3,3-trifluorpropyl)amino]piperidin-4-carboxamid;
- 1-{[4-(4-Chlorphenyl)-5-(2,4-dichlorphenyl)-3-(3-hydroxypropoxy)thien-2-yl]carbonyl}-4-[(3,3,3-trifluorpropyl)amino]piperidin-4-carboxamid;
- 1-{[4-(4-Chlorphenyl)-5-(2,4-dichlorphenyl)-3-{2-[(hydroxyacetyl)amino]ethoxy}thien-2-yl]carbonyl}-3-phenylpiperidin-4-carboxamid;
- 1-{[3-(2-Acetamidoethoxy)-4-(4-chlorphenyl)-5-(2,4-dichlorphenyl)thien-2-yl]carbonyl}-4-phenylpiperidin-4-carboxamid;
- 1-{[4-(4-Chlorphenyl)-5-(2,4-dichlorphenyl)-3-(2-hydroxyethoxy)thien-2-yl]carbonyl}-4-[(4-fluorbenzyl)amino]piperidin-4-carboxamid;
- 1-{[4-(4-Chlorphenyl)-5-(2,4-dichlorphenyl)-3-(3-hydroxypropoxy)thien-2-yl]carbonyl}-4-[(4-fluorbenzyl)amino]piperidin-4-carboxamid;
- 1-({4-(4-Chlorphenyl)-5-(2,4-dichlorphenyl)-3-[3-(methylsulfonyl)propoxy]thien-2-yl}carbonyl)-4-[(4-fluorbenzyl)amino]piperidin-4-carboxamid;
- 1-{[3-(2-Acetamidoethoxy)-4-(4-chlorphenyl)-5-(2,4-dichlorphenyl)thien-2-yl]carbonyl}-4-[(4-fluorbenzyl)amino]piperidin-4-carboxamid;
- 1-{[4-(4-Chlorphenyl)-5-(2,4-dichlorphenyl)-3-{2-[(hydroxyacetyl)amino]ethoxy}thien-2-yl]carbonyl}-4-[(4-fluorbenzyl)amino]piperidin-4-carboxamid;
- 1-{[4-(4-Chlorphenyl)-5-(2,4-dichlorphenyl)-3-{3-[(hydroxyacetyl)amino]propoxy}thien-2-yl]carbonyl}-4-[(4-fluorbenzyl)amino]piperidin-4-carboxamid;
- 1-{[3-(3-Acetamidopropoxy)-4-(4-chlorphenyl)-5-(2,4-dichlorphenyl)thien-2-yl]carbonyl}-4-[(4-fluorbenzyl)amino]piperidin-4-carboxamid;
- 4-(Benzylamino)-1-{[4-(4-chlorphenyl)-5-(2,4-dichlorphenyl)-3-(2-hydroxyethoxy)thien-2-yl]carbonyl}piperidin-4-carboxamid;
- 4-(Benzylamino)-1-{[4-(4-chlorphenyl)-5-(2,4-dichlorphenyl)-3-{2-[(hydroxyacetyl)amino]ethoxy}thien-2-yl]carbonyl}piperidin-4-carboxamid;
- 1-{[5-(2-Chlorphenyl)-4-(4-chlorphenyl)-3-(3-hydroxypropoxy)thien-2-yl]carbonyl}-4-[(4-fluorbenzyl)amino]piperidin-4-carboxamid;
- 1-{[5-(2-Chlorphenyl)-4-(4-chlorphenyl)-3-(3-hydroxypropoxy)thien-2-yl]carbonyl}-4-phenylpiperidin-4-carboxamid;
- 1-{[5-(2-Chlorphenyl)-4-(4-chlorphenyl)-3-(2-hydroxyethoxy)thien-2-yl]carbonyl}-4-phenylpiperidin-4-carboxamid;
- 1-{[5-(2-Chlorphenyl)-4-(4-chlorphenyl)-3-(2-hydroxyethoxy)thien-2-yl]carbonyl}-4-[(4-fluorbenzyl)amino]piperidin-4-carboxamid;
- 1-{[3-(2-Aminoethoxy)-5-(2-chlorphenyl)-4-(4-chlorphenyl)thien-2-yl]carbonyl}-4-[(4-fluorbenzyl)amino]piperidin-4-carboxamid;
- 1-{[3-(2-Acetamidoethoxy)-5-(2-chlorphenyl)-4-(4-chlorphenyl)thien-2-yl]carbonyl}-4-[(4-fluorbenzyl)amino]piperidin-4-carboxamid;
- 1-{[3-(2-Aminoethoxy)-5-(2-chlorphenyl)-4-(4-chlorphenyl)thien-2-yl]carbonyl}-4-phenylpiperidin-4-carboxamid;
- 1-{[5-(2-Chlorphenyl)-4-(4-chlorphenyl)-3-{2-[(hydroxyacetyl)amino]ethoxy}thien-2-yl]carbonyl}-4-phenylpiperidin-4-carboxamid;
- 1-{[3-(2-Acetamidoethoxy)-5-(2-chlorphenyl)-4-(4-chlorphenyl)thien-2-yl]carbonyl}-4-phenylpiperidin-4-carboxamid;
- 1-{[5-(2-Chlorphenyl)-4-(4-chlorphenyl)-3-{2-(hydroxyacetyl)amino]ethoxy}thien-2-yl]carbonyl}-4-[(4-fluorbenzyl)amino]piperidin-4-carboxamid;

in Form einer Base oder eines Säureadditionssalzes.

5. Verfahren zur Herstellung einer Verbindung der Formel (I) nach Anspruch 1, **dadurch gekennzeichnet, dass**:

in Gegenwart einer Base eine Verbindung der Formel:

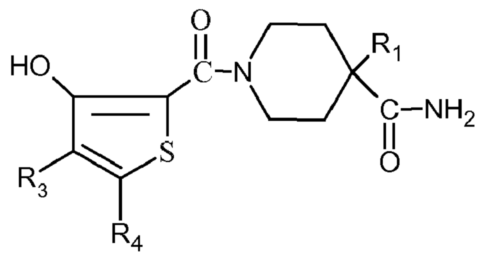

(II)

worin $R_1$, $R_3$ und $R_4$ wie für eine Verbindung der Formel (I) nach Anspruch 1 definiert sind, mit einer Verbindung der Formel:

$Z-R_2$ (III)

worin $R_2$ wie für eine Verbindung der Formel (I) nach Anspruch 1 definiert ist, und Z für eine Abgangsgruppe steht, umgesetzt wird.

6. Verfahren zur Herstellung einer Verbindung der Formel (I) nach Anspruch 1, **dadurch gekennzeichnet, dass**:

eine Säure oder ein funktionelles Derivat dieser Säure der Formel:

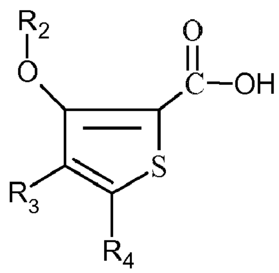

(IV)

worin $R_2$, $R_3$ und $R_4$ wie für eine Verbindung der Formel (I) nach Anspruch 1 definiert sind, mit einer Verbindung der Formel:

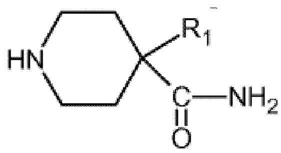

(V)

worin $R_1$ so wie für eine Verbindung der Formel (I) nach Anspruch 1 definiert ist, umgesetzt wird.

7. Medikament, **dadurch gekennzeichnet, dass** es eine Verbindung der Formel (I) nach einem der Ansprüche 1 bis 4 oder ein Säureadditionssalz dieser Verbindung der Formel (I) mit einer pharmazeutisch verträglichen Säure umfasst.

8. Pharmazeutische Zusammensetzung, **dadurch gekennzeichnet, dass** sie eine Verbindung der Formel (I) nach einem der Ansprüche 1 bis 4 oder ein Säureadditionssalz dieser Verbindung der Formel (I) mit einer pharmazeutisch verträglichen Säure sowie mindestens einen pharmazeutisch verträglichen Exzipienten umfasst.

9. Verwendung einer Verbindung nach einem der Ansprüche 1 bis 4 zur Herstellung eines Medikaments, das zur Behandlung und/oder Prävention von psychiatrischen Störungen, Substanzabhängigkeit und -entwöhnung, kognitiven Störungen, Aufmerksamkeits- und Wachsamkeitsstörungen, akuten und chronischen neurodegenerativen Erkrankungen bestimmt ist.

10. Verwendung einer Verbindung nach einem der Ansprüche 1 bis 4 zur Herstellung eines Medikaments, das zur

Behandlung und/oder Prävention von Stoffwechselstörungen, Appetitstörungen, Appetenzstörungen, Fettleibigkeit, Bulimie, Diabetes, metabolischem Syndrom, Dyslipidämie bestimmt ist.

**11.** Verwendung einer Verbindung nach einem der Ansprüche 1 bis 4 zur Herstellung eines Medikaments, das zur Behandlung und/oder Prävention von Schmerz, neuropathischen Schmerzen, akuten peripheren Schmerzen, chronischen Schmerzen entzündlichen Ursprungs, durch Antikrebsbehandlung induzierten Schmerzen, bestimmt ist.

**12.** Verwendung einer Verbindung nach einem der Ansprüche 1 bis 4 zur Herstellung eines Medikaments, das zur Behandlung und/oder Prävention von Magen-Darm-Störungen, Erbrechen, Durchfallstörungen, Geschwüren, Lebererkrankungen bestimmt ist.

**13.** Verwendung einer Verbindung nach einem der Ansprüche 1 bis 4 zur Herstellung eines Medikaments, das zur Behandlung und/oder Prävention von entzündlichen Erscheinungen, Erkrankungen des Immunsystems, rheumatoider Arthritis, Erkrankungen, die zu einer Demyelinisierung führen, multipler Sklerose, Knochenerkrankungen und Osteoporose bestimmt ist.

**14.** Verwendung einer Verbindung nach einem der Ansprüche 1 bis 4 zur Herstellung eines Medikaments, das zur Behandlung und/oder Prävention von Bewegungserkrankungen und-störungen, insbesondere Dyskinesien oder Parkinson-Krankheit, senilen Demenzen und Alzheimer-Krankheit bestimmt ist.

## Claims

**1.** Compound of formula (I):

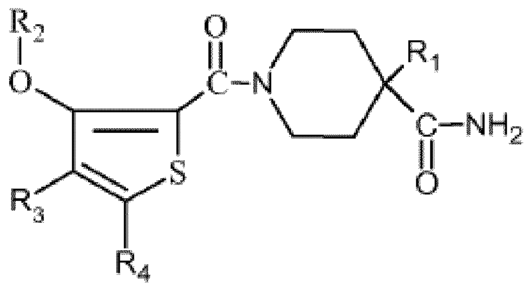

(I)

in which:

- $R_1$ represents:
    - · an $-NR_5R_6$ group;
    - · a phenyl which is unsubstituted or substituted one or more times with substituents chosen independently from a halogen atom, a $(C_1-C_4)$alkyl, a $(C_1-C_4)$alkoxy and a trifluoromethyl;
- $R_2$ represents:
    - · a $(C_1-C_4)$alkyl;
    - · an $-X-R_7$ group;
- $R_3$ and $R_4$ each independently represent a phenyl substituted one or more times with a substituent chosen independently from a halogen atom, a $(C_1-C_4)$alkyl, a $(C_1-C_4)$alkoxy and a trifluoromethyl;
- $R_5$ represents a hydrogen atom or a $(C_1-C_4)$alkyl;
- $R_6$ represents a $(C_1-C_4)$alkyl which is unsubstituted or substituted with one or more fluorine atoms or with a phenyl which is unsubstituted or substituted with a halogen atom;
- or else $R_5$ and $R_6$, together with the nitrogen atom to which they are bonded, constitute a heterocycle chosen from: azetidine, pyrrolidine, and piperidine, said heterocycle being unsubstituted or substituted one or more times with a halogen atom;
- X represents a $(C_1-C_5)$alkylene;

- $R_7$ represents an $-OR_8$ group, an $-NR_9R_{10}$ group or an $-SO_2-(C_1-C_4)$alkyl group;
- $R_8$ represents a hydrogen atom or a $(C_1-C_4)$alkyl;
- $R_9$ represents a hydrogen atom or a $(C_1-C_4)$alkyl;
- $R_{10}$ represents a hydrogen atom, a $-COR_{11}$ group, an $-SO_2R_{11}$ group or a $-CO(CH_2)_mOH$ group;
- $R_{11}$ represents a $(C_1-C_4)$alkyl which is unsubstituted or substituted with one or more fluorine atoms;
- m represents 1, 2 or 3;

in the form of a base or of an addition salt with an acid.

**2.** Compound of formula (I), according to Claim 1, in which:

- $R_1$ represents:

  · an $-NR_5R_6$ group;
  · a phenyl;

- $R_2$ represents:

  · a methyl;
  · an $-X-R_7$ group;

- $R_3$ and $R_4$ each independently represent a phenyl substituted once or twice with a halogen atom;
- $R_5$ represents a hydrogen atom;
- $R_6$ represents a $(C_1-C_4)$alkyl substituted with one or more fluorine atoms or with a phenyl which is unsubstituted or substituted with a halogen atom;
- or else $R_5$ and $R_6$, together with the nitrogen atom to which they are bonded, constitute a piperidin-1-yl radical which is unsubstituted or substituted once or twice with a halogen atom;
- X represents a $(C_1-C_3)$alkylene;
- $R_7$ represents an $-OR_8$ group, an $-NR_9R_{10}$ group or an $-SO_2-CH_3$ group;
- $R_8$ represents a hydrogen atom;
- $R_9$ represents a hydrogen atom;
- $R_{10}$ represents a hydrogen atom, a $-CO-CH_3$ group, an $-SO_2-CH_3$ group or a $-COCH_2OH$ group;

in the form of a base or of an addition salt with an acid.

**3.** Compound of formula (I), according to Claim 1, in which:

- $R_1$ represents:

  · an $-NH(CH_2)_2-CF_3$ group, a 4-fluorobenzylamino group, a benzylamino group, a piperidin-1-yl radical, a 4,4-difluoropiperidin-1-yl radical;
  · a phenyl;

- $R_2$ represents:

  · a methyl;
  · an $-X-R_7$ group;

- $R_3$ represents a 4-chlorophenyl;
- $R_4$ represents a 2-chlorophenyl or a 2,4-dichlorophenyl;
- X represents a methylene, an ethylene or a trimethylene;
- $R_7$ represents an -OH radical, an $-NH_2$ radical, an $-NHCOCH_3$ group, an $-NHSO_2CH_3$ group, an $-NHCOCH_2OH$ group or an $-SO_2-CH_3$ group;

in the form of a base or of an addition salt with an acid.

**4.** Compound according to Claim 1, chosen from:

- 1'-{[4-(4-chlorophenyl)-5-(2,4-dichlorophenyl)-3-methoxythien-2-yl]carbonyl}-1,4'-bipiperidine-4'-carboxamide;
- 1'-{[4-(4-chlorophenyl)-5-(2,4-dichlorophenyl)-3-{3-[(methylsulfonyl)amino]propoxy}thien-2-yl]carbonyl}-1,4'-bipiperidine-4'-carboxamide;
- 1'-({4-(4-chlorophenyl)-5-(2,4-dichlorophenyl)-3-[3-(methylsulfonyl)propoxy]thien-2-yl}carbonyl)-1,4'-bipiperidine-4'-carboxamide;
- 1-{[4-(4-chlorophenyl)-5-(2,4-dichlorophenyl)-3-(3-hydroxypropoxy)thien-2-yl]carbonyl}-4-phenylpiperidine-4-carboxamide;
- 1-({4-(4-chlorophenyl)-5-(2,4-dichlorophenyl)-3-[3-(methylsulfonyl)propoxy]thien-2-yl}carbonyl)-4-phenylpiperidine-4-carboxamide;
- 1-{[4-(4-chlorophenyl)-5-(2,4-dichlorophenyl)-3-(2-hydroxyethoxy)thien-2-yl]carbonyl}-4-phenylpiperidine-4-carboxamide;
- 1'-{[4-(4-chlorophenyl)-5-(2,4-dichlorophenyl)-3-(2-hydroxyethoxy)thien-2-yl]carbonyl}-4,4-difluoro-1,4'-bipiperidine-4'-carboxamide;
- 1'-{[4-(4-chlorophenyl)-5-(2,4-dichlorophenyl)-3-(3-hydroxypropoxy)thien-2-yl]carbonyl}-4,4-difluoro-1,4'-bipiperidine-4'-carboxamide;
- 1-{[4-(4-chlorophenyl)-5-(2,4-dichlorophenyl)-3-(2-hydroxyethoxy)thien-2-yl]carbonyl}-4-[(3,3,3-trifluoropropyl)amino]piperidine-4-carboxamide;
- 1-({4-(4-chlorophenyl)-5-(2,4-dichlorophenyl)-3-[3-(methylsulfonyl)propoxy]thien-2-yl}carbonyl)-4-[(3,3,3-trifluoropropyl)amino]piperidine-4-carboxamide;
- 1-{[4-(4-chlorophenyl)-5-(2,4-dichlorophenyl)-3-{2-(hydroxyacetyl)amino]ethoxy}thien-2-yl]carbonyl}-4-[(3,3,3-trifluoropropyl)amino]piperidine-4-carboxamide;
- 1-{[4-(4-chlorophenyl)-5-(2,4-dichlorophenyl)-3-(3-hydroxypropoxy)thien-2-yl]carbonyl}-4-[(3,3,3-trifluoropropyl)amino]piperidine-4-carboxamide;
- 1-{[4-(4-chlorophenyl)-5-(2,4-dichlorophenyl)-3-{2-[(hydroxyacetyl)amino]ethoxy}thien-2-yl]carbonyl}-3-phenylpiperidine-4-carboxamide;
- 1-{[3-(2-acetamidoethoxy)-4-(4-chlorophenyl)-5-(2,4-dichlorophenyl)thien-2-yl]carbonyl}-4-phenylpiperidine-4-carboxamide;
- 1-{[4-(4-chlorophenyl)-5-(2,4-dichlorophenyl)-3-(2-hydroxyethoxy)thien-2-yl]carbonyl}-4-[(4-fluorobenzyl)amino]piperidine-4-carboxamide;
- 1-{[4-(4-chlorophenyl)-5-(2,4-dichlorophenyl)-3-(3-hydroxypropoxy)thien-2-yl]carbonyl}-4-[(4-fluorobenzyl)amino]piperidine-4-carboxamide;
- 1-({4-(4-chlorophenyl)-5-(2,4-dichlorophenyl)-3-[3-(methylsulfonyl)propoxy]thien-2-yl}carbonyl)-4-[(4-fluorobenzyl)amino]piperidine-4-carboxamide;
- 1-{[3-(2-acetamidoethoxy)-4-(4-chlorophenyl)-5-(2,4-dichlorophenyl)thien-2-yl]carbonyl}-4-[(4-fluorobenzyl)amino]piperidine-4-carboxamide;
- 1-{[4-(4-chlorophenyl)-5-(2,4-dichlorophenyl)-3-{2-[(hydroxyacetyl)amino]ethoxy}thien-2-yl]carbonyl}-4-[(4-fluorobenzyl)amino]piperidine-4-carboxamide;
- 1-{[4-(4-chlorophenyl)-5-(2,4-dichlorophenyl)-3-{3-[(hydroxyacetyl)amino]propoxy}thien-2-yl]carbonyl}-4-[(4-fluorobenzyl)amino]piperidine-4-carboxamide;
- 1-{[3-(3-acetamidopropoxy)-4-(4-chlorophenyl)-5-(2,4-dichlorophenyl)thien-2-yl]carbonyl}-4-[(4-fluorobenzyl)amino]piperidine-4-carboxamide;
- 4-(benzylamino)-1-{[4-(4-chlorophenyl)-5-(2,4-dichlorophenyl)-3-(2-hydroxyethoxy)thien-2-yl]carbonyl}piperidine-4-carboxamide;
- 4-(benzylamino)-1-{[4-(4-chlorophenyl)-5-(2,4-dichlorophenyl)-3-{2-[(hydroxyacetyl)amino]ethoxy}thien-2-yl]carbonyl}piperidine-4-carboxamide;
- 1-{[5-(2-chlorophenyl)-4-(4-chlorophenyl)-3-(3-hydroxypropoxy)thien-2-yl]carbonyl}-4-[(4-fluorobenzyl)amino]piperidine-4-carboxamide;
- 1-{[5-(2-chlorophenyl)-4-(4-chlorophenyl)-3-(3-hydroxypropoxy)thien-2-yl]carbonyl}-4-phenylpiperidine-4-carboxamide;
- 1-{[5-(2-chlorophenyl)-4-(4-chlorophenyl)-3-(2-hydroxyethoxy)thien-2-yl]carbonyl}-4-phenylpiperidine-4-carboxamide;
- 1-{[5-(2-chlorophenyl)-4-(4-chlorophenyl)-3-(2-hydroxyethoxy)thien-2-yl]carbonyl}-4-[(4-fluorobenzyl)amino]piperidine-4-carboxamide;
- 1-{[3-(2-aminoethoxy)-5-(2-chlorophenyl)-4-(4-chlorophenyl)thien-2-yl]carbonyl}-4-[(4-fluorobenzyl)amino]piperidine-4-carboxamide;
- 1-{[3-(2-acetamidoethoxy)-5-(2-chlorophenyl)-4-(4-chlorophenyl)thien-2-yl]carbonyl}-4-[(4-fluorobenzyl)amino]piperidine-4-carboxamide;

- 1-{[3-(2-aminoethoxy)-5-(2-chlorophenyl)-4-(4-chlorophenyl)thien-2-yl]carbonyl}-4-phenylpiperidine-4-carboxamide;
- 1-{[5-(2-chlorophenyl)-4-(4-chlorophenyl)-3-{2-[(hydroxyacetyl)amino]ethoxy}thien-2-yl]carbonyl}-4-phenylpiperidine-4-carboxamide;
- 1-{[3-(2-acetamidoethoxy)-5-(2-chlorophenyl)-4-(4-chlorophenyl)thien-2-yl]carbonyl}-4-phenylpiperidine-4-carboxamide;
- 1-{[5-(2-chlorophenyl)-4-(4-chlorophenyl)-3-{2-(hydroxyacetyl)amino]ethoxy}thien-2-yl]carbonyl}-4-[(4-fluorobenzyl)amino]piperidine-4-carboxamide; in the form of a base or of an addition salt with an acid.

5. Process for preparing a compound of formula (I) according to Claim 1, **characterized in that**:

a compound of formula:

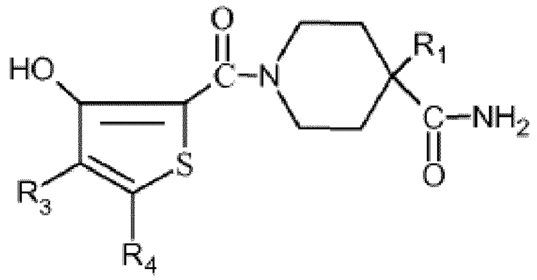

(II)

in which $R_1$, $R_3$ and $R_4$ are as defined for a compound of formula (I) according to Claim 1, is reacted, in the presence of a base, with a compound of formula:

$Z\text{-}R_2$ (III)

in which $R_2$ is as defined for a compound of formula (I) according to Claim 1 and Z represents a leaving group.

6. Process for preparing a compound of formula (I) according to Claim 1, **characterized in that**:

an acid or a functional derivative of this acid of formula:

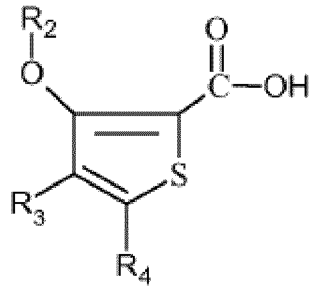

(IV)

in which $R_2$, $R_3$ and $R_4$ are as defined for a compound of formula (I) according to Claim 1, is reacted with a compound of formula:

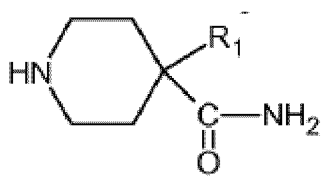

(V)

in which $R_1$ is as defined for a compound of formula (I) according to Claim 1.

7. Medicament, **characterized in that** it comprises a compound of formula (I) according to any one of Claims 1 to 4, or an addition salt of this compound of formula (I) with a pharmaceutically acceptable acid.

8. Pharmaceutical composition, **characterized in that** it comprises a compound of formula (I) according to any one of Claims 1 to 4, or an addition salt of this compound of formula (I) with a pharmaceutically acceptable acid, and also at least one pharmaceutically acceptable excipient.

9. Use of a compound according to any one of Claims 1 to 4, for the preparation of a medicament for use in the treatment and/or prevention of psychiatric disorders, dependence on and withdrawal from a substance, cognitive disorders, attention and consciousness disorders, and acute and chronic neurodegenerative diseases.

10. Use of a compound according to any one of Claims 1 to 4, for the preparation of a medicament for use in the treatment and/or prevention of metabolic disorders, appetite disorders, appetence disorders, obesity, bulimia, diabetes, metabolic syndrome and dyslipidemia.

11. Use of a compound according to any one of Claims 1 to 4, for the preparation of a medicament for use in the treatment and/or prevention of pain, neuropathic pain, acute peripheral pain, chronic pain of inflammatory origin, and pain induced by an anticancer treatment.

12. Use of a compound according to any one of Claims 1 to 4, for the preparation of a medicament for use in the treatment and/or prevention of gastrointestinal disorders, vomiting, diarrhea disorders, ulcers and liver diseases.

13. Use of a compound according to any one of Claims 1 to 4, for the preparation of a medicament for use in the treatment and/or prevention of inflammatory phenomena, immune system diseases, rheumatoid arthritis, diseases resulting in demyelination, multiple sclerosis, bone diseases and osteoporosis.

14. Use of a compound according to any one of Claims 1 to 4, for the preparation of a medicament for use in the treatment and/or prevention of motor diseases and disorders, in particular dyskinesia or Parkinson's disease, senile dementia and Alzheimer's disease.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5624941 A
- EP 0576357 A
- EP 0656354 A
- EP 1150961 A
- WO 03051850 A
- WO 03027076 A
- WO 9119708 A
- WO 2005035488 A
- WO 2006070106 A
- WO 2006077320 A
- WO 2006084975 A
- EP 0428434 A
- EP 0512901 A
- EP 0515240 A
- WO 9623787 A
- WO 02094821 A
- WO 03104225 A
- WO 2006021654 A


### Littérature non-brevet citée dans la description


- **GREEN et al.** Protective Group in Organic Synthesis. John Wiley & Sons, Inc, 2007
- **M. B. SMITH ; J. MARCH.** Advanced Organic Chemistry. Wiley Interscience, 2007, 496-501
- *J. Med. Chem.,* 1964, vol. 7, 619-622
- *J. Org. Chem.,* 1990, vol. 55, 4207-4209
- **M. RINALDI-CARMONA et al.** *FEBS Letters,* 1994, vol. 350, 240-244
- **M. BOUABOULA et al.** *J. Biol. Chem.,* 1995, vol. 270, 13973-13980
- **M. RINALDI-CARMONA et al.** *J. Pharmacol. Exp. Ther.,* 1996, vol. 278, 871-878
- **M. BOUABOULA et al.** *J. Biol. Chem.,* 1997, vol. 272, 22330-22339
- **M. RINALDI-CARMONA et al.** *Life Sciences,* 1995, vol. 56, 1941-1947
- **M. RINALDI-CARMONA et al.** *J. Pharmacol. Exp. Ther.,* 2004, vol. 310, 905-914
- **RINALDI-CARMONA M. et al.** *JPET,* 2004, vol. 310, 905-914